# EUROPEAN PATENT SPECIFICATION

(11) **EP 2 413 145 B1**
(45) Date of publication and mention of the grant of the patent: **14.04.2021**
(21) Application number: 11174488.4
(22) Date of filing: 19.07.2011
(51) Int. Cl.: G01N 35/00, G16H 40/40, G16H 10/40

(54) **Clinical sample analyzing system, clinical sample analyzer, management apparatus, and method of managing clinical sample analyzer**
Klinisches Probeanalysesystem, klinisches Probenanalysegerät, Verwaltungsverfahren und Verfahren zur Verwaltung des klinischen Probenanalysegerätes
Système d'analyse d'échantillons cliniques, analyseur d'échantillons cliniques, appareil de gestion et procédé de gestion d'analyseur d'échantillons cliniques

(30) Priority: 27.07.2010 JP 2010167725
(43) Date of publication of application: 01.02.2012
(73) Proprietor: SYSMEX CORPORATION, Kobe-shi, Hyogo 651-0073 (JP)
(72) Inventor: Shindo, Naoki, Hyogo, 651-0073 (JP); Sone, Atsumasa, Hyogo, 651-0073 (JP); Kishida, Taizo, Hyogo, 651-0073 (JP); Hirata, Tsukasa, Hyogo, 651-0073 (JP)
(74) Representative: Hoffmann Eitle

(56) References cited:
- EP-A2- 1 835 291
- WO-A1-02/052278
- US-A1- 2004 078 219
- US-A1- 2005 254 999
- US-A1- 2007 027 635
- US-A1- 2010 005 344

## Description

### FIELD OF THE INVENTION

The present invention relates to a clinical sample analyzing system which includes a clinical sample analyzer and a management apparatus which provides information about maintenance on the clinical sample analyzer to technicians who perform maintenance on the clinical sample analyzer, the clinical sample analyzer, the management apparatus, and a method of managing the clinical sample analyzer.

### BACKGROUND

There have been known systems which are used in maintenance on sample analyzers in support centers. In U.S. Patent 6, 629, 060, a remote support system is disclosed which includes a sample analyzer and a management apparatus which is connected to the sample analyzer via a communication network. In such a remote support system disclosed in USP 6,629,060, when an error such as a failure or a malfunction occurs in the sample analyzer, the sample analyzer transmits error information in real time. In addition, the sample analyzer transmits operation information with no urgency such as the number of operations and the sample measurement result at the time of shutdown. The error information and the operation information are received by the management apparatus and registered in a database. In the support center, technicians monitor the error information and the operation information of the sample analyzer on the management apparatus, and when an abnormality occurs in the sample analyzer, the technicians perform repair and maintenance on the sample analyzer.

In order to perform rapid repair and maintenance when a failure or a malfunction occurs in the sample analyzer, when error information is transmitted, technicians are required to rapidly confirm the transmitted error information.

Accordingly, in the conventional system, support center technicians are required to stand by before the management apparatus in order to cope with error information where the transmission time is unknown regardless of the schedules of sample measurement by the sample analyzer, and thus a burden is imposed on the technicians.

The invention is contrived in view of such circumstances and a main object thereof is to provide a sample analyzing system which can reduce the burden on an operator and allow rapid maintenance of the sample analyzer, the sample analyzer, a management apparatus, and a method of managing the sample analyzer.

US 2007/027635 A1 describes an external quality control method, using an external quality control system which comprises an external quality control computer and a plurality of preprocessing devices connected to the external quality control computer via a network. The method comprises: performing preprocessing operations on preprocessing quality control samples, the preprocessing devices being adapted to prepare samples for measurement of target nucleic acid; measuring preprocessed preprocessing quality control samples to obtain measurement data; providing the measurement data to the external quality control computer over the network; storing the measurement data; and implementing an external quality control process based on the measurement data. This document also describes an external quality control method for detection of nucleic acid, an external quality control method for preparation of calibration curve, an external quality control computer, a preprocessing device, and a nucleic acid detecting device.

US 2004/078219 A1 describes a healthcare network for sharing information concerning the health of a user with at least one outside source, the network including a biosensor associated with the user that generates a biosensor signal containing the information; and a personal data control means including receiving means for receiving the biosensor signal, input means for receiving a privacy input from the user, and output means for generating a response signal based on the biosensor signal and privacy input. The network also includes a data allocation and processing module including means for receiving the response signal, and means for generating and directing an output signal to the at least one outside source, wherein the module is responsive to the response signal, and wherein the availability of the information to the at least one outside source is responsive to the privacy input.

US 2010/0005344 A1 describes a networked clinical diagnostic analyzer with support for monitoring by a remote back office. The analyzer includes a module to detect in real-time events of interest while allowing the back office to modify the definitions of the events. The module may be networked using virtual private networking into the back office network, which is usually different from the network at the site of the clinical diagnostic analyzer's deployment. The real-time alerts are said to allow both a quick response to actual or expected error conditions and the downloading of logged data that is likely of most interest and relevance.

US 2005/0254999 A1 describes clinical specimen processing apparatuses and clinical specimen processing systems which are connectable to a management apparatus over a network.

### SUMMARY OF THE PRESENT INVENTION

In order to solve the above-described problem, a first aspect of the present invention is a clinical sample analyzing system comprising: a clinical sample analyzer configured to analyze a clinical sample, wherein the clinical sample analyzer is to be installed in a facility of an user; and a management apparatus which is able to perform data communication with the clinical sample analyzer and is to be installed in a facility different from the facility of the user, wherein the clinical sample analyzer is configured to automatically transmit report data showing an operation situation of the clinical sample analyzer to the management apparatus when a predetermined event related to an initiation of a sample measurement occurs, and the management apparatus is configured to output a notification when receiving the report data from the clinical sample analyzer.

In this aspect, when a first event related to an initiation of a sample measurement occurs, the clinical sample analyzer may transmits first report data showing the occurrence of the first event to the management apparatus, and when a second event related to an initiation of a sample measurement occurs after the occurrence of the first event, the clinical sample analyzer may transmits second report data showing the occurrence of the second event to the management apparatus.

In this aspect, the predetermined event may include at least one of: an event related to a measurement of a patient sample; an event related to the start-up of the clinical sample analyzer; an event related to the measurement of a standard sample for preparation of a calibration curve or accuracy management; and an event related to the approval for a prepared calibration curve or an accuracy management result.

In this aspect, the clinical sample analyzer may transmit the report data showing an operation situation of the clinical sample analyzer when the predetermined event occurs, and the management apparatus may outputs a notification of the operation situation of the clinical sample analyzer when receiving the report data.

In this aspect, the clinical sample analyzer may automatically transmit a measurement completion report for reporting a completion of a sample measurement to the management apparatus when the sample measurement is completed, and the management apparatus may output a notification of the completion of a sample measurement when receiving the measurement completion report.

In this aspect, the clinical sample analyzer may automatically transmit an operation termination report for reporting a termination of the operation of the clinical sample analyzer to the management apparatus when executing a termination operation of the clinical sample analyzer, and the management apparatus may output a notification of the termination of the operation of the clinical sample analyzer when receiving the operation termination report.

In this aspect, the clinical sample analyzer may transmit an analysis result to the management apparatus when completing the analysis of the clinical sample, and the management apparatus may be able to output the received analysis result.

In this aspect, the clinical sample analyzer may automatically transmit abnormality report data showing an abnormality to the management apparatus when the abnormality occurs in the clinical sample analyzer, and the management apparatus may output the occurrence of the abnormality in the clinical sample analyzer when receiving the abnormality report data.

In this aspect, the abnormality report data may include information showing the content of the abnormality occurring in the clinical sample analyzer, and the management apparatus may output the information showing the content of the abnormality in the clinical sample analyzer when receiving the abnormality report data.

In this aspect, after output of the notification, the management apparatus may receive a confirmation input from a person receiving the notification, and when receiving a confirmation input, record a reception of a confirmation input.

In this aspect, the management apparatus may be connected to a phone line, and when a predetermined time elapses from the execution of the notification without reception of the confirmation input, the management apparatus may perform a phone call to a predetermined phone number in order to prompt the confirmation of the notification.

In this aspect, the clinical sample analyzer may be configured to amplify a target nucleic acid in a tissue collected from a patient and detects an amplified target nucleic acid.

A second aspect of the present invention is a clinical sample analyzer which is to be installed in a facility of an user, the analyzer comprising: means for performing data communication with a management apparatus which is to be installed in a facility different from the facility of the user, wherein the means for performing data communication is configured to automatically transmit report data showing an operation situation of the clinical sample analyzer to a management apparatus when a predetermined event related to an initiation of a sample measurement occurs.

A third aspect of the present invention is a management apparatus which is to be installed in a facility different from a facility of an user in which a clinical sample analyzer is to be installed, the management apparatus capable of performing data communication with the clinical sample analyzer and comprising: means for receiving, when a predetermined event related to an initiation of a sample measurement occurs, report data showing an operation situation of the clinical sample analyzer which is transmitted from the clinical sample analyzer in order to report the occurrence of the predetermined event; and means for outputting a notification of the operation situation of the clinical sample analyzer when the report data is received by the receiving section.

A fourth aspect of the present invention is a method of managing a clinical sample analyzer, the method comprising: a transmitting step of automatically transmitting report data showing an operation situation of the clinical sample analyzer by the clinical sample analyzer when a predetermined event related to an initiation of a sample measurement occurs in the clinical sample analyzer which is installed in a facility of an user; and an output step of outputting a notification of the operation situation of the clinical sample analyzer when a management apparatus receives the report data, wherein the management apparatus is able to perform data communication with the clinical sample analyzer and is installed in a facility different from the facility of an user.

### ADVANTAGE OF THE INVENTION

According to the present inventions, an operator can know that the sample measurement by the sample analyzer may be executed on the basis of the notification by the management apparatus. Accordingly, the operator can stand by before the management apparatus to cope with a failure or a malfunction, and thus can perform rapid repair and maintenance. Furthermore, the operator is needed to stand by only after a notification is outputted. Therefore, the burden on an operator can be reduced.

### BRIEF DESCRIPTION OF THE DRAWINGS

[Fig. 1] Fig. 1 is a perspective view showing the configuration of a sample analyzing system according to an embodiment.
[Fig. 2] Fig. 2 is a perspective view showing the configuration of a sample analyzer according to the embodiment.
[Fig. 3] Fig. 3 is a perspective view showing the configuration of a preprocessing unit.
[Fig. 4] Fig. 4 is a perspective view showing the configuration of a measuring unit.
[Fig. 5] Fig. 5 is a plan view showing the configuration of the measuring unit.
[Fig. 6] Fig. 6 is a block diagram showing the configuration of a data processing unit.
[Fig. 7] Fig. 7 is a graph showing the relationship between an amplification rise time and a concentration.
[Fig. 8] Fig. 8 is a calibration curve graph showing the relationship between an amplification rise time and the number of copies of a target gene.
[Fig. 9] Fig. 9 is a schematic view showing the configuration of a database.
[Fig. 10] Fig. 10 is a block diagram showing the configuration of a management server according to the embodiment.
[Fig. 11] Fig. 11 is a flowchart showing the operation procedure of the sample analyzer according to the embodiment.
[Fig. 12] Fig. 12 is a flowchart showing the error notification operation procedure of the sample analyzer.
[Fig. 13] Fig. 13 is a flowchart showing the operation procedure of the management server.
[Fig. 14] Fig. 14 is a diagram showing an example of a monitoring screen.
[Fig. 15] Fig. 15 is a diagram showing an example of a reception situation screen.

### PREFERRED EMBODIMENT

Hereinafter, preferred embodiments of the invention will be described with reference to the drawings.

### [Configuration of Sample Analyzing System]

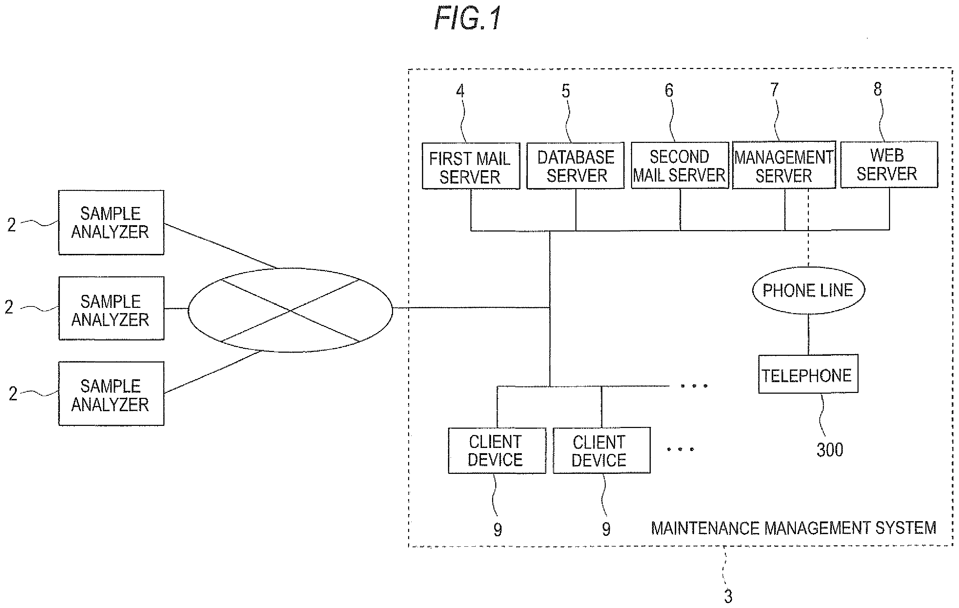

Fig. 1 is a perspective view showing the configuration of a sample analyzing system 1 according to this embodiment. The sample analyzing system 1 includes sample analyzers 2, 2, ... which are installed in a user facility such as a hospital or an examination center and a maintenance management system 3 which is installed in a facility for maintenance service providers such as a maker of the sample analyzers 2 which performs maintenance on the sample analyzers 2. The sample analyzers 2, 2, ... are connected to the maintenance management system 3 so as to perform data communication therewith via a communication network such as the Internet or a dedicated line. The maintenance management system 3 includes a first mail server 4, a database server 5, a second mail server 6, a management server 7, a web server 8, and client devices 9, 9, .... The first mail server 4, the database server 5, the second mail server 6, the management server 7, the web server 8, and the client devices 9, 9, ... are connected so as to perform data communication with each other by LAN. In addition, the management server 7 which is connected to a phone line can make a call to a telephone 300 of the person in charge of maintenance service and can outputs a predetermined voice message during telephone communication.

### <Sample Analyzer>

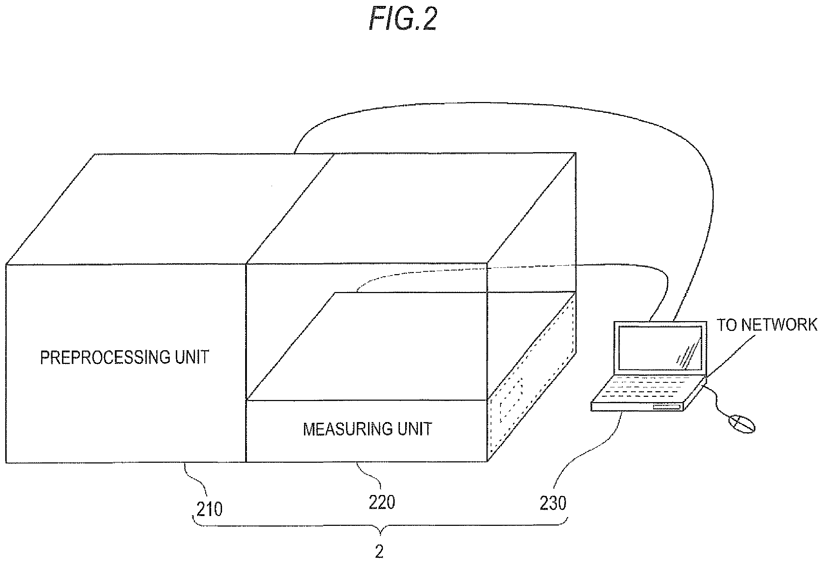

Fig. 2 is a perspective view showing the configuration of the sample analyzer 2. The sample analyzer 2 according to this embodiment is a nucleic acid amplification detector which sets a cut tissue from a living body (human body), such as a lymph node, as a sample and can output the concentration of a target nucleic acid (target gene) included in this sample as measurement data. In greater detail, this sample analyzer 2 which is used as a genetic diagnosis system of breast cancer lymph node metastasis performs preprocessing (homogenization, extraction treatment, and the like) on the lymph node (sample) cut from a human body to prepare a solubilized extract which is a sample for measurement for nucleic acid detection and amplifies the target nucleic acid (target gene) present in the sample for measurement by a Loop-mediated Isothermal Amplification (LAMP) method to measure the turbidity of the solution which occurs with the amplification, thereby obtaining the concentration of the target nucleic acid (oncogene; mRNA).

This sample analyzer 2 is used for intraoperative rapid diagnosis, and in greater detail, the analyzer is used during the operation for a breast cancer and the like. For example, the sample analyzer 2 obtains, from the lymph node which is cut intraoperatively, the concentration of cancer-derived genes (target nucleic acid) in the lymph node to allow a doctor to diagnose the degree of cancer metastasis intraoperatively on the basis of this concentration, and a lymph node dissection range is determined. Accordingly, high reliability and promptness are required for the output of the sample analyzer 2.

As shown in Fig. 2, the sample analyzer 2 has a preprocessing unit 210 for preparing a sample for measurement by performing preprocessing such as homogenization on a sample which is obtained from a human body or the like and a measuring unit 220 which performs a process of detecting a target nucleic acid included in the sample for measurement. In addition, the sample analyzer 2 has a data processing unit 230 for performing data processing, data communication and the like. This data processing unit 230 also functions as a controller which receives measurement data from the preprocessing unit 210 and the measuring unit 220 or transmits an operation instruction signal and the like to the preprocessing unit 210 and the measuring unit 220. That is, the preprocessing unit 210 and the data processing unit 230 function as a preprocessor, and the measuring unit 220 and the data processing unit 230 function as a nucleic acid detector. In addition, the data processing unit 230 is connected to a network and can transmit the measurement data and the like transmitted from a transmitting section of each of the preprocessing unit 210 and the measuring unit 220 to the maintenance management system 3 by a transmission-reception function between the above-described maintenance management system 3 and the data processing unit 230.

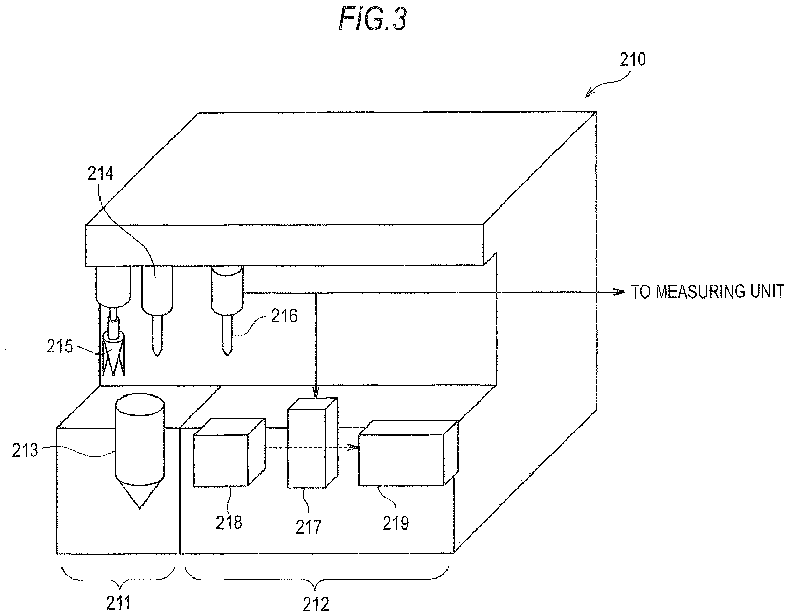

Fig. 3 is a perspective view showing the configuration of the preprocessing unit 210. As shown in Fig. 3, the preprocessing unit 210 mainly includes a preprocessing section 211 which performs preprocessing on a sample to prepare a sample for measurement and a measuring section 212 which measures a sample for measurement on which the preprocessing has been completed. The preprocessing section 211 includes a sample setting section 213 which sets a container containing a sample, a reagent adding section (reagent dispensing pipette) 214 which adds a reagent for preprocessing to a container which is set in the sample setting section 213 and contains a sample, a blender (homogenization section) 215 for performing sample homogenization, a pipette (dispensing section) 216 which dispenses a homogenized (preprocessed) sample for measurement, and a transfer section (not shown) which transfers the pipette 216 to the measuring section 212 and the measuring unit 220.

When receiving a measurement start instruction signal from the data processing unit 230, the preprocessor adds a reagent for preprocessing to a sample of the sample setting section 213 (the process of adding a reagent for preprocessing), homogenizes the sample by the blender 215, and prepares a sample for measurement (homogenization process). The pipette 216 suctions the sample for measurement (hereinafter, also referred to as "sample"), and in the case of normal nucleic acid detection, the pipette 216 moves up to the measuring unit 220 and injects the sample into a sample container 22 set in the measuring unit 220.

Meanwhile, in the case of accuracy management, the pipette 216, which has suctioned an accuracy management sample for measurement prepared by performing preprocessing on an accuracy management sample for preprocessing, moves to an absorbance measurement cell 217 and injects the accuracy management sample for measurement into the absorbance measurement cell 217 of the measuring section 212. The absorbance measurement cell 217 is irradiated with light from an optical source 218, the light is detected by a detector (light-receiving section) 219, and the absorbance of the preprocessed sample is measured. The measured absorbance (measurement data) is transmitted to the data processing unit 230 by a transmitting section (omitted in the drawing) of the preprocessing unit 210. The preprocessing is not limited to homogenization and may be a nucleic acid extraction process or the like.

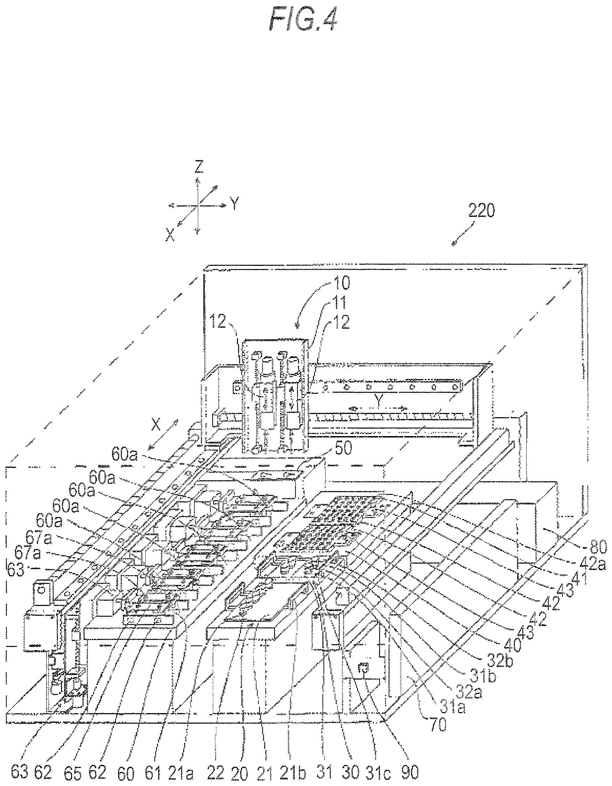

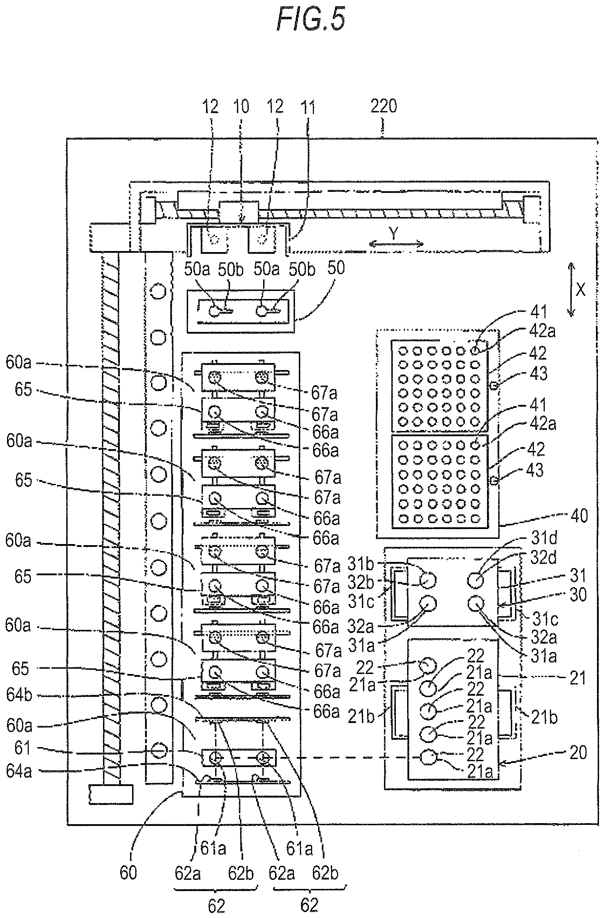

Fig. 4 is a perspective view showing the configuration of the measuring unit 220, and Fig. 5 is a plan view showing the configuration of the measuring unit 220. The measuring unit 220 is configured as shown in Figs. 4 and 5 and described in detail in JP-A-2005-98960. Here, the configuration, operation and the like of the measuring unit 220 will be briefly described. First, the pipette moved from the preprocessing unit 210 injects the preprocessed sample into the sample container 22 set in a sample container setting hole 21a of a sample container table 21.

In a primer reagent container setting hole 31a and an enzyme reagent container setting hole 31b on the front-left side of a reagent container setting section 30, a primer reagent container 32a containing a primer reagent of CK19 (cytokeratin 19) and an enzyme reagent container 32b containing an enzyme reagent are set, respectively. In addition, in a primer reagent container setting hole 31a on the front-right side of the reagent container setting section 30, a primer reagent container 32a containing a primer reagent of Arabidopsis (hereinafter, referred to as arabido) which is an internal standard material is set. In addition, in an arabido container setting hole 31d on the front-right side, an arabido solution container 32d containing a predetermined amount of arabido is set.

In addition, two racks 42, each having 36 disposable pipette chips 41 stored therein, are fitted in concave sections (not shown) of a chip setting section 40. Furthermore, two cell sections 66a of a detection cell 65 are set in two detection cell setting holes of a reaction section 61 of each reaction detection block 60a.

In this state, when the operation of the measuring unit 220 is started, first, an arm section 11 of a dispensing mechanism section 10 is moved to the chip setting section 40 from the initial position, and then two syringe sections 12 of the dispensing mechanism section 10 is moved downward in the chip setting section 40. In this manner, the tip ends of the nozzle sections of the two syringe sections 12 are pushed into upper opening sections of the two pipette chips 41, and thus the pipette chips 41 are automatically mounted on the tip ends of the nozzle sections of the two syringe sections 12. The two syringe sections 12 are moved upward, and then the arm section 11 of the dispensing mechanism section 10 is moved in the X-axis direction toward the upper part of the two primer reagent containers 32a which contain primer reagents of CK19 and arabido, respectively, and are set in a reagent container setting table 31. In addition, due to the downward movement of the two syringe sections 12, the tip ends of the two pipette chips 41 mounted on the nozzle sections of the two syringe sections 12 are inserted into the liquid surfaces of the primer regents of CK19 and arabido in the two primer reagent containers 32a, respectively. The primer regents of CK19 and arabido in the two primer reagent containers 32a are suctioned by pump sections of the syringe sections 12.

The two syringe sections 12 are moved upward after suction of the primer reagents, and then the arm section 11 of the dispensing mechanism section 10 is moved to the upper part of the reaction detection block 60a which is positioned on the innermost side (on the innermost side from the front of the apparatus). In this case, the arm section 11 of the dispensing mechanism section 10 is moved so as not to pass over the other second to fifth reaction detection blocks 60a. In addition, due to the downward movement of the two syringe sections 12 in the reaction detection block 60a on the innermost side, the two pipette chips 41 mounted on the nozzle sections 12a of the two syringe sections 12 are inserted into the two cell sections 66a of the detection cell 65. In addition, using the pump sections of the syringe sections 12, the two primer reagents of CK19 and arabido are discharged to the two cell sections 66a, respectively (primer reagent dispensing process).

Thereafter, the pipette chips 41 are destroyed and two new pipette chips 41 are automatically mounted on the tip ends of the nozzle sections of the two syringe sections 12. Then, the enzyme reagent in the enzyme reagent container 32b is discharged to the two cell sections 66a of the detection cell 65 (the process of dispensing an enzyme reagent) with almost the same operation as in the above description. Thereafter, the arabido solution in the arabido solution container 32d is discharged to the two cell sections 66a of the detection cell 65 in the same manner. Thereafter, the sample (sample for measurement) in the sample container 22 is discharged to the two cell sections 66a of the detection cell 65 in the same manner (the process of dispensing a sample). In this manner, the specimen for detecting CK19 is adjusted in one cell section 66a of the detection cell 65, and the specimen for detecting arabido is adjusted in the other cell section 66a.

After the discharge of the primer reagent, enzyme reagent, arabido solution and sample into the cell sections, a cap closing operation of the detection cell 65 is performed. After completion of the cap closing operation, the liquid temperature in the detection cell 65 is increased to about 65°C from about 20°C by using a Peltier module of the reaction section 61 to amplify the target gene (CK19) and the arabido in accordance with the LAMP method. The white turbidity caused by magnesium pyrophosphate which is formed with the amplification is detected by a turbidimetric method. In greater detail, the cell section 66a of the detection cell 65 at the time of amplification reaction is irradiated with light having a diameter of about 1 mm from an LED optical source section 62a of a turbidity detection section 62 via an optical irradiation groove of the reaction section 61. The emitted light is received by a photodiode light-receiving section 62b. In this manner, the liquid turbidity in the cell section 66a of the detection cell 65 at the time of amplification reaction is detected (monitored) in real time. The measurement data of CK19 and the measurement data of arabido measured by the photodiode light-receiving section 62b are transmitted to the data processing unit 230 by a transmitting section (not shown) of the measuring unit 220.

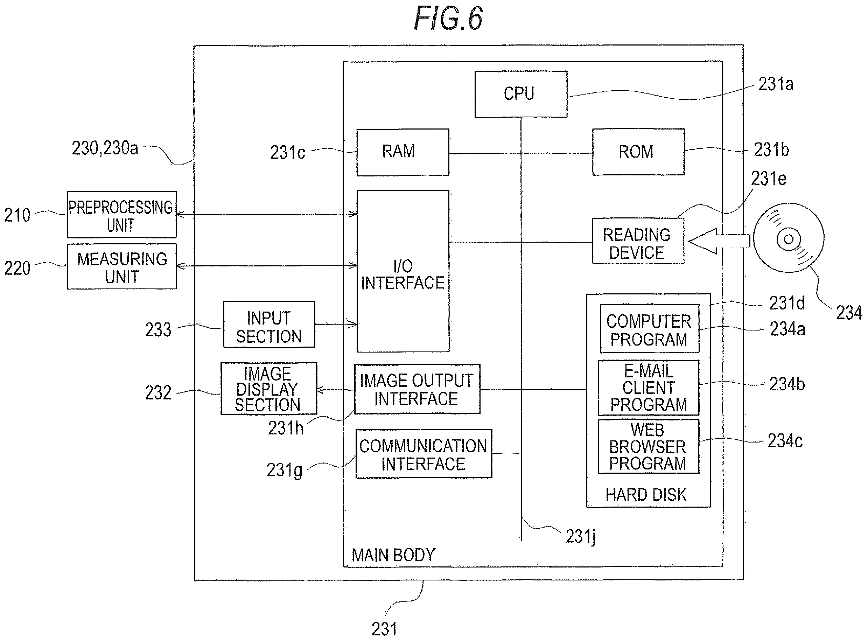

Next, the configuration of the data processing unit 230 will be described. Fig. 6 is a block diagram showing the configuration of the data processing unit 230. The data processing unit 230 is realized by a computer 230a. As shown in Fig. 6, the computer 230a includes a main body 231, an image display section 232, and an input section 233. The main body 231 includes a CPU 231a, a ROM 231b, a RAM 231c, a hard disk 231d, a reading device 231e, an I/O interface 231f, a communication interface 231g, and an image output interface 231h, and the CPU 231a, the ROM 231b, the RAM 231c, the hard disk 231d, the read-out device 231e, the I/O interface 231f, the communication interface 231g, and the image output interface 231h are connected to each other by a bus 231j.

The read-out device 231e reads out a computer program 234a for allowing the computer to function as the information processing unit 230 from a portable recording medium 234 and can install the computer program 234a in the hard disk 231d.

In addition, an e-mail client program 234b is installed in the hard disk 231d. When such an e-mail client program 234b is executed by the CPU 231a, the data processing unit 230 functions as a client of the e-mail system and can transmit an e-mail.

Furthermore, a web browser program 234c is installed in the hard disk 231d. When such a web browser program 234c is executed by the CPU 231a, the data processing unit 230 functions as a web client, can receive HTML data transmitted from the web server, and can display a web page on the image display section 232.

The I/O interface 231f is connected to the preprocessing unit 210 and the measuring unit 220 via a cable. The I/O interface 231f is connected to the preprocessing unit 210 and the measuring unit 220 so as to perform data communication therewith, and can output a control signal to the preprocessing unit 210 and the measuring unit 220. The control sections (not shown) of the preprocessing unit 210 and the measuring unit 220 receiving such a control signal decode this control signal, and in response to the control signal, the actuators of the mechanism sections are driven. In addition, the measurement data can be transmitted to the data processing unit 230 from each of the preprocessing unit 210 and the measuring unit 220, and when the data processing unit 230 receives the measurement data, the CPU 231a performs a predetermined process on the measurement data.

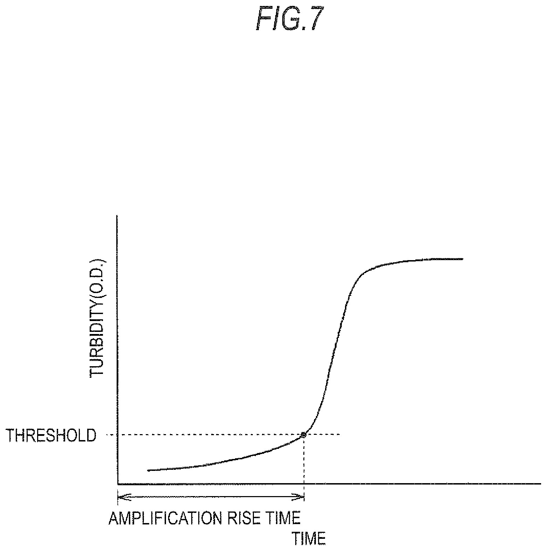

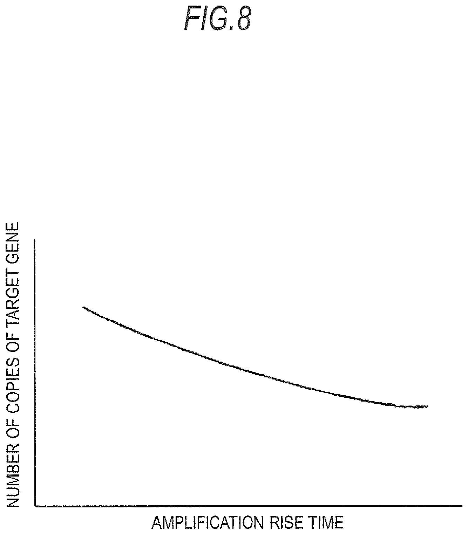

The process by the data processing unit 230 on the measurement data of the measuring unit 220 will be further described in detail. As described above, the measurement data of CK19 and the measurement data of arabido measured by the photodiode light-receiving section 62b are transmitted from the measuring unit 220. In the data processing unit 230, when the horizontal axis represents time and the vertical axis represents turbidity (O.D.: Optical Density) , the measurement data of CK19 is obtained as shown in Fig. 7. In addition, the data processing unit 230 detects an amplification rise time, which is a time until the number of copies of the target gene (CK19) in the sample rapidly increases from this measurement data of CK19, by comparing the turbidity with a predetermined threshold. Meanwhile, in the same manner as above, the data processing unit 230 prepares measurement data of arabido with the horizontal axis representing time and the vertical axis representing turbidity from the measurement data of arabido, and acquires an amplification rise time of arabido on the basis of the measurement data. The data processing unit 230 corrects the amplification rise time of CK19 on the basis of this amplification rise time of arabido. Due to such correction, the effect of the amplification inhibitor in the sample on the measurement result can be removed. In addition, on the basis of the calibration curve which is prepared from the measurement result of the calibrator of CK19 in advance as shown in Fig. 8, the amount (number of copies) of the target gene (CK19) is calculated from the corrected amplification rise time of CK19. Here, the calibration curve shown in Fig. 8 is a curve with the horizontal axis representing an amplification rise time and the vertical axis representing the number of copies [number of copies/µL] of the target gene (CK19) . In general, the shorter the amplification rise time is, the higher the concentration of the target genes is.

The data of the calculated amount of the target gene is displayed on the screen by a display device of the data processing unit 230 or another display device. In addition, the data processing unit 230 obtains a qualitative determination result for diagnosis support from the quantitative measurement data (amplification rise time, the number of copies) and displays the qualitative determination result on the screen by the display device of the data processor or another display device. Regarding this determination, for example, the case in which the number of copies is 250 or less, or the case in which the turbidity does not reach a threshold even when a predetermined time elapses in the measurement data shown in Fig. 7 is determined as "ND", the case in which the number of copies is in the range of 250 to 5×10³ is determined as "+", and the case in which the number of copies is greater than 5×10³ is determined as "++". Here, cancer metastasis degrees are qualitatively shown, such as "ND" representing "no metastasis is detected", "+" representing "little metastasis", and "++" representing that "metastasis is evident", and when the sample analyzer 2 obtains and displays qualitative results to be helpful in support for the accurate diagnosis from the quantitative measurement data (number of cancer-derived cells), a doctor rapidly makes a diagnosis intraoperatively and can determine a dissection range.

In addition, the sample analyzing system 1 is connected to various sample analyzers such as a blood cell counter, a blood coagulation measurement apparatus, an immunoassay apparatus, a biochemical analyzer, a urine qualitative analyzer, and an in-urine physical component analyzer, other than the above-described nucleic acid amplification detector.

### <First Mail Server>

The first mail server 4 is realized by a computer. Since the configuration of the computer realizing such a first mail server 4 is the same as the configuration of the computer 230a realizing the data processing unit 230, the description thereof will be omitted.

A mail server program is installed on the hard disk of the computer constituting the first mail server 4. The computer functions as the first mail server when the CPU of the computer executes the mail server program. The e-mail transmitted from the data processing unit 230 is received by the first mail server 4 and stored in the mail box which is provided in the hard disk of the first mail server 4.

### <Database Server>

The database server 5 is realized by a computer. Since the configuration of the computer realizing such a database server 5 is the same as the configuration of the computer 230a realizing the data processing unit 230, the description thereof will be omitted.

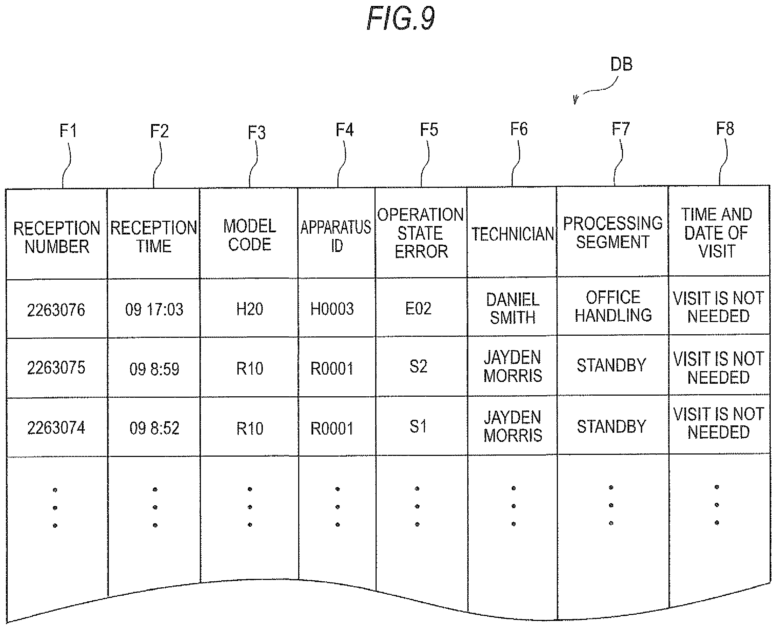

The hard disk of the computer constituting the database server 5 is provided with a database for storing state information related to the state of the sample analyzers 2, 2, .... Fig. 9 is a schematic view showing the configuration of the database. A database DB is provided with a field F1 for storing the number (receipt number) of the received data, a field F2 for storing the data reception time, a field F3 for storing the model code of the sample analyzer, a field F4 for storing the apparatus ID assigned to each sample analyzer, a field F5 for storing the operation state code showing the state of the apparatus or the error code showing the type of the error of the apparatus, a field F6 for storing the name of a technician who performed data updating, a field F7 for storing the data processing segment, and a field F8 for storing the time and date of the visit of the worker to the facility. In addition, the computer functions as the database sever 5 when the CPU of the computer executes a database server program which is installed on the hard disk of the computer. When an e-mail related to the apparatus state transmitted from the sample analyzer 2 is received by the first mail server 4, the information which is included in the e-mail is acquired by the database server 5 and stored in the database DB. When the information which is stored in this manner in the database DB is information (hereinafter, referred to as "operation report information") which reports the execution of an event related to the start of sample measurement of the sample analyzer or urgent error information, the database server 5 creates and transmits an e-mail including the operation report information or the urgent error information to the second mail server 6. The event related to the start of sample measurement includes at least one of (1) an event related to the measurement of a patient sample, (2) an event related to the start-up of the sample analyzer, (3) an event related to the measurement of a standard sample for preparation of a calibration curve or accuracy management, and (4) an event related to the approval for a prepared calibration curve or the accuracy management result. In this embodiment, (1) the event related to the measurement of a patient sample is that the start of measurement of a patient sample is received from a user via the input section 233 of the data processing unit 230. (2) The event related to the start-up of the sample analyzer is a shift to a standby state of the measuring unit 220. (3) The event related to the measurement of a standard sample for preparation of a calibration curve or accuracy management is that the start of the measurement of the calibrator is received from a user via the input section 233 of the data processing unit 230. (4) The event related to the approval for a prepared calibration curve or the accuracy management result is that a validation for the calibration curve is received from a user via the input section 233 of the data processing unit 230. The events will be described later in detail.

In addition, the database server 5 is configured to store the accuracy management result data transmitted from the sample analyzer 2 in an accuracy management database and store the measurement data transmitted from the sample analyzer 2 in a measurement result database. The database server 5 transmits the operation report information, the urgent error information, the accuracy management result data, and the measurement data stored in the databases tc the web server 8 and the information can be viewed from the computers such as the data processing unit 230 and the client devices 9 by the web server 8.

### <Second Mail Server>

The second mail server 6 is realized by a computer. Since the configuration of the computer realizing such a second mail server 6 is the same as the configuration of the computer 230a realizing the data processing unit 230, the description thereof will be omitted.

A mail server program is installed on the hard disk of the computer constituting the second mail server 6. The computer functions as the second mail server when the CPU of the computer executes the mail server program. The e-mail transmitted from the database server 5 is received by the second mail server 6 and stored in the mail box which is provided in the hard disk of the second mail server 6.

### <Management Server>

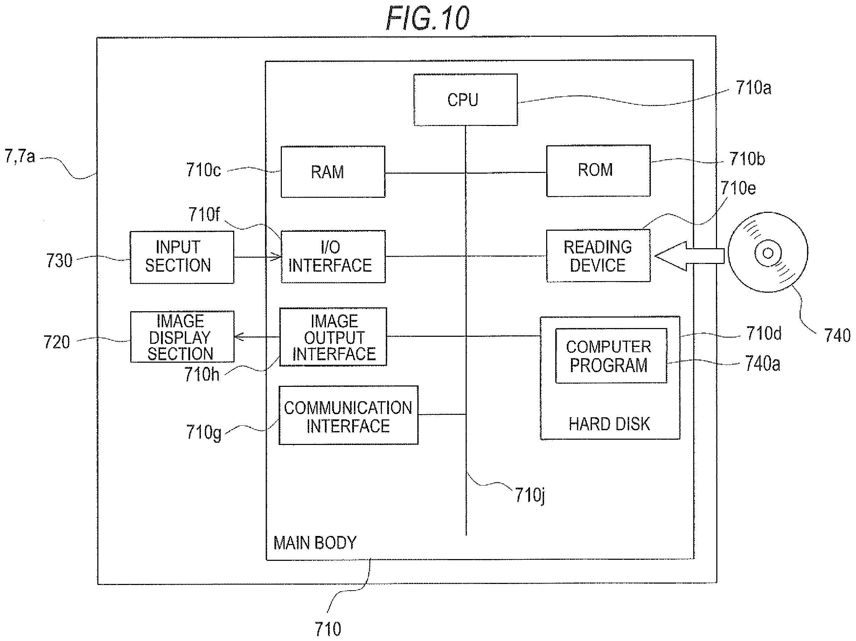

Fig. 10 is a block diagram showing the configuration of the management server. The management server 7 is realized by a computer 7a. As shown in Fig. 10, the computer 7a includes a main body 710, an image display section 720, and an input section 730. The main body 710 includes a CPU 710a, a ROM 710b, a RAM 710c, a hard disk 710d, a reading device 710e, an I/O interface 710f, a communication interface 710g, and an image output interface 710h, and the CPU 710a, the ROM 710b, the RAM 710c, the hard disk 710d, the read-out device 710e, the I/O interface 710f, the communication interface 710g, and the image output interface 710h are connected to each other by a bus 710j.

The reading device 710e can read out a computer program 740a for allowing the computer to function as the management server 7 from a portable recording medium 740 to install the computer program 740a on the hard disk 710d.

When an e-mail including the operation report information or the urgent error information is received by the second mail server 6, the management server 7 transmits the operation report information or the urgent error information to each of the client devices 9, 9, ... in order to notify a technician of the reception of the operation report information or the urgent error information.

### <Web Server 8>

The web server 8 is realized by a computer. Since the configuration of the computer realizing such a web server 8 is the same as the configuration of the computer 230a realizing the data processing unit 230, the description thereof will be omitted.

The web server 8 receives and stores the measurement result and the accuracy management result data transmitted from the database server 5 in the hard disk. When a request for viewing the information is received from the computers such as the data processing unit 230 and the client devices 9, HTML data including the measurement result or the accuracy management result data is transmitted to the computer which is a request source. In this manner, the information can be viewed by the computers such as the data processing unit 230 and the client devices 9.

### <Client Device>

The client device 9 is realized by a computer. Since the configuration of the computer realizing such a client device 9 is the same as the configuration of the computer 230a realizing the data processing unit 230, the description thereof will be omitted.

A management client program is installed on the hard disk of the computer functioning as the client device 9. The computer functions as the client device when the CPU of the computer executes the management client program. The client device 9 can access the management server 7, is notified of the fact that the sample analyzer 2 is operating or the fact that an urgent error has occurred in the sample analyzer 2 from the management server 7, and displays the information on the image display section. In addition, a web browser program is installed on the hard disk of the client device 9. The client device 9 receives the measurement result and the accuracy management result of the sample analyzer 2 by accessing the web server 8 and can display a web page including the measurement result and the accuracy management result of the sample analyzer 2 on the image display section.

### [Operation of Sample Analyzing System]

### <Operation of Sample Analyzer>

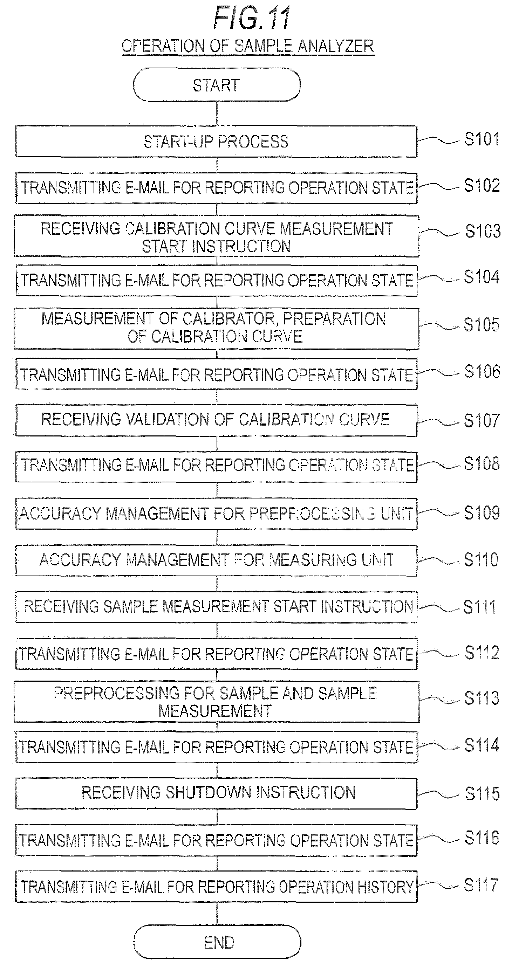

Hereinafter, the operation of the sample analyzing system 1 according to this embodiment will be described. Fig. 11 is a flowchart showing the operation procedure of the sample analyzer 2 according to this embodiment. The sample analyzer 2 is used to analyze a sample (lymph node) which is collected during the operation for a breast cancer and the like and is started-up before the operation (Step S101). The start-up process is executed as follows. The measuring unit 220 of the sample analyzer 2 is provided with a power button (not shown), and the measuring unit 220 is turned on when a user presses the power button. When the unit is turned on, the measuring unit 220 executes the adjustment of the original point and the confirmation of the operation of the mechanism section and shifts to a standby state. In this manner, the start-up process is completed. When detecting the shift of the measuring unit 220 to the standby state, the CPU 231a of the data processing unit 230 generates and transmits an e-mail for notifying of the start-up of the sample analyzer 2 to the first server (Step S102).

Here, the above-described e-mail will be described. The destination of this e-mail is a mail address for providing maintenance service, and the e-mail includes the model code of the sample analyzer, the apparatus ID of the sample analyzer, and the operation state code of the sample analyzer in the subject thereof. In addition, the body of the e-mail is left blank.

The hard disk 231d of the data processing unit 230 stores the model code and the serial number of the sample analyzer 2. The operation state codes are as follows. "0" represents a state in which the sample analyzer is started-up (start-up state), "1" represents a state in which the measurement of the calibrator is started to prepare the calibration curve (calibration curve measurement start state), "2" represents a state in which the measurement of the calibrator ends to prepare the calibration curve (calibration curve measurement end state), "3" represents a state in which the prepared calibration curve is approved by a user (calibration curve validation state), "4" represents a state in which the sample measurement is started (sample measurement start state), "5" represents a state in which the sample measurement ends (sample measurement end state), and "6" represents a state in which the sample analyzer (measuring unit) is shut down (measuring unit end state). In the above-described Step S102, the CPU 231a of the data processing unit 230 generates an e-mail, of which the destination is a mail address for providing maintenance service stored in the hard disk 231d and which includes the model code and the serial number stored in the hard disk 231d and the operation state code (in this case, "0") corresponding to the state of the apparatus at that time in the subject thereof.

Next, the calibration curve to be used in the sample analysis is prepared. The calibration curve is prepared through the measurement of the calibrator by the measuring unit 220. The calibrator includes a predetermined amount of CK19 which is a target nucleic acid, and three kinds of calibrators each having a different amount of CK19 are used.

The sample containers 22 accommodating these calibrators are set in the sample container table 21 of the measuring unit 220 before the calibration curve preparation process. A user inputs a start instruction by the input section 233 of the data processing unit 230 in order to start the calibration curve preparation process (calibrator measurement process) of the measuring unit 220. When receiving such an instruction for starting the calibration curve measurement (Step S103), the CPU 231a generates and transmits an e-mail for notifying of the start of the calibration curve measurement to the first mail server (Step S104). In the subject of this e-mail, the operation state code "1", showing the state in which the calibration curve measurement is started, is included. Thereafter, the sample analyzer 2 executes the measurement of the calibrator and the CPU 231a prepares the calibration curve (Step S105).

The process in Step S105 will be described in detail. When receiving a signal of the measurement start instruction, the measuring unit 220 subjects each of the three calibrators to the primer reagent dispensing process, the enzyme reagent dispensing process, and the calibrator solution dispensing process of dispensing the calibrator of the sample container 22 into one cell section 66a of the detection cell 65. Thereafter, the measuring unit 220 increases the liquid temperature in the detection cell 65 to about 65°C from about 20°C to amplify the target nucleic acid by the LAMP (nucleic acid amplification) reaction, and performs a detection process of detecting the liquid turbidity in the cell section 66a of the detection cell 65 at the time of amplification reaction by the turbidity detection section 62.

The measuring unit 220 transmits the detected optical information (measurement data) to the data processing unit 230. When receiving the optical information (liquid turbidity) of each calibrator from the measuring unit 220, the data processing unit 230 performs a process of analyzing the optical information. In the analysis process, the amplification rise time of each calibrator is calculated. The amplification rise time is calculated as a time until the liquid turbidity obtained as the optical information exceeds a predetermined value. The data processing unit 230 prepares a new calibration curve from the amplification rise time calculated with respect to each calibrator on the basis of the calibration curve which is being kept or the number of copies of the indicated value of each calibrator, and calculates the number of copies of CK19 of each calibrator.

After preparation of the calibration curve, the CPU 231a generates and transmits an e-mail for notifying of the end of the calibration curve measurement to the first mail server (Step S106). In the subject of this e-mail, the operation state code "2", showing the state in which the calibration curve measurement ends, is included.

The prepared calibration curve is displayed on the image display section 232 of the data processing unit 230. The data processing unit 230 can receive a validation for the calibration curve from a user. The user confirms the calibration curve displayed on the image display section 232, and validates the calibration curve if the calibration curve has no abnormality. When receiving the validation for the calibration curve (Step S107), the CPU 231a generates and transmits an e-mail for notifying of the validation for the calibration curve to the first mail server (Step S108). In the subject of this e-mail, the operation state code "3", showing the state in which the calibration curve is validated, is included.

The above-described rise time of CK19 and number of copies of CK19 (measurement data) are transmitted to the database server 5 from the data processing unit 230. The measurement data includes information such as the apparatus ID of the sample analyzer which measures the calibrator, the lot number of the calibrator, and the measurement time other than the rise time and the number of copies of CK19.

When receiving the analysis result (measurement data), the database server 5 accumulates the measurement data in the accuracy management database. In addition, the database server 5 subjects a large number of pieces of measurement data transmitted from the sample analyzers which are a large number of nucleic acid examination systems which are installed in the respective facilities to a statistical process. In greater detail, on the basis of the measurement data transmitted from the sample analyzers 2 (data processing unit 230) which are installed in a plurality of facilities, the average value for each day and the standard deviation 1SD are obtained for each measurement item. In addition, the database server 5 also obtains 2SD, which is two times the standard deviation 1SD, and 3SD, which is three times the standard deviation 1SD. The average value of the measurement data for each day, 1SD, 2SD, and 3SD are accumulated in an accuracy management statistical database of the database server 5. In addition, the measurement data of a reference apparatus which is a reference sample analyzer is also accumulated in the accuracy management statistical database.

Furthermore, when receiving the measurement data, the database server 5 determines whether or not the calibration curve preparation process is normal on the basis of the calculated average value and 1SD, 2SD or 3SD. Each of the 1SD, the 2SD, and the 3SD can be a reference value for whether or not the received measurement data is normal. Which one of the 1SD, the 2SD, and the 3SD is used as the reference value is selected by each facility and the selected reference value is used in determination. Such a determination result is also registered in the accuracy management database.

When the accuracy management data (measurement data, statistical data and determination result) of the calibrator is registered in the accuracy management database, the database server 5 transmits the registered accuracy management data to the web server 8. The web server 8 stores the received accuracy management data in the database in the hard disk. Such accuracy management data can be viewed from the other computers (data processing unit 230 and client devices 9) by the web server 8.

Next, a user prompts the sample analyzer 2 to manage the accuracy of the preprocessing unit 210 (Step S109). In the external accuracy management of the preprocessing unit 210, a sample for accuracy management (accuracy management sample for preprocessing) is preprocessed by the preprocessing unit 210 to prepare an accuracy management sample for measurement, and the absorbance of the accuracy management sample for measurement is measured. The accuracy management sample for preprocessing is constituted as a false tissue which includes a known amount of target nucleic acid or cells including the known amount of target nucleic acid and a holder capable of holding the target nucleic acid or the cells including the target nucleic acid. This false tissue is prepared so that a predetermined reference value (indicated value) is obtained when the preprocessing unit 210 performs predetermined preprocessing and the absorbance is measured.

The nucleic acid which is used in the false tissue may be not only DNA or RNA, but also an artificial nucleic acid such as PNA, BNA or an analog thereof. The cell which is used in the false tissue is not particularly limited if it is a cell containing the target nucleic acid. The holder preferably has a solid form at room temperatures and preferably flows with the collapsing solid form thereof due to a temperature increase up to a certain temperature. In addition, the holder in the solid form preferably has the same level of hardness as the body tissue.

The holder preferably includes a gelator. The gelator is a substance having properties of gelating the solution by being added to a solvent. Examples of the gelator include natural polymers such as agar, agarose, carrageenan, alginic acid, alginate, pectin, collagen, gelatin and gluten, synthetic polymers such as polyvinyl alcohol (PVA), polyethylene glycol (PEG) and polyacrylamide (PAA), and the like. As the false tissue of this embodiment, one or two or more kinds can be used among these synthetic polymers and natural polymers. The solvent to which the gelator is added is not particularly limited. However, for example, water, Tris EDTA (TE), Tris-Acetate EDTA (TAE) , Tris-Borate EDTA (TBE) and the like can be used.

The external accuracy management is executed once or several times a day, and normal sample measurement is performed after the external accuracy management. First, in the accuracy management, the false tissue (accuracy management sample for preprocessing) is set in the sample setting section 213 of the preprocessing unit 210. When a user inputs a start instruction by the input section 233 of the data processing unit 230 of the sample analyzer 2 in order to start the external accuracy management of the preprocessing unit 210 and the data processing unit 230 receives the instruction, the data processing unit 230 transmits a measurement start instruction for preprocessing to the preprocessing unit 210.

When receiving a signal of the measurement start instruction, the preprocessing unit 210 performs the process of adding a reagent for preprocessing and the homogenization process on an accuracy management sample for preprocessing by the preprocessing section 211 to prepare an accuracy management sample for measurement. This accuracy management sample for measurement is given to the measuring section 212 of the preprocessing unit 210 and the absorbance is measured. The absorbance measurement data is transmitted to the first mail server 4 by the data processing unit 230.

When receiving the absorbance measurement data, the first mail server 4 transmits the absorbance measurement data to the database server 5 and the measurement data is registered in the accuracy management database. In addition, the database server 5 subjects a large number of pieces of absorbance measurement data transmitted from a large number of the sample analyzers 2 which are installed in the respective facilities to the statistical process. In greater detail, on the basis of the absorbance measurement data transmitted from the sample analyzers 2 which are installed in a plurality of facilities, the average value for each day and the standard deviation 1SD are obtained. In addition, the database server 5 also obtains 2SD, which is two times the standard deviation 1SD, and 3SD, which is three times the standard deviation 1SD. The average value of the absorbance measurement data for each day, 1SD, 2SD, and 3SD are registered in the accuracy management database of the database server 5. In the accuracy management database, the absorbance measurement data which is obtained by measuring the false tissue preprocessed by a reference apparatus (reference sample analyzer) is also accumulated.

Furthermore, when receiving the absorbance measurement data, the database server 5 determines whether or not the preprocessing by the preprocessor is normal on the basis of the calculated average value and 1SD, 2SD or 3SD. In greater detail, the database server 5 determines whether or not the preprocessing is normal on the basis of the average value of the absorbance measurement data which was received over a certain period of time (for example, 24 hours in the past) and the standard deviation 1SD, 2SD or 3SD. Each of the 1SD, the 2SD, and the 3SD can be a reference value for whether or not the received absorbance measurement data is normal. Which one of the 1SD, the 2SD, and the 3SD is used as the reference value is selected by each facility and the selected reference value is used in determination. Such a determination result is also registered in the accuracy management database.

When the accuracy management data (measurement data, statistical data and determination result) is registered in the accuracy management database, the database server 5 transmits the registered accuracy management data to the web server 8. The web server 8 stores the received accuracy management data in the database in the hard disk. Such accuracy management data can be viewed from the other computers (data processing unit 230 and client devices 9) by the web server 8.

Next, a user prompts the sample analyzer 2 to manage the accuracy of the measuring unit 220 (Step S110) . In the external accuracy management of the measuring unit 220, in place of a normal sample for measurement, an accuracy management sample for nucleic acid detection (hereinafter, also simply referred to as "control solution") is measured by the measuring unit 220. As the control solution, two kinds, that is, CK19 control (first accuracy management substance for nucleic acid detection) containing a known amount of CK19 which is a target nucleic acid and lacking arabido which is an internal standard nucleic acid (nucleic acid derived from plant; absent in human body) and Internal control (arabido control; second accuracy management substance for nucleic acid detection) containing a known amount of arabido which is an internal standard nucleic acid and lacking CK19 which is a target nucleic acid.

First, prior the external accuracy management (control solution measurement process), a sample container 22 containing CK19 control and a sample container 22 containing arabido control are set in the sample container table 21 of the measuring unit 220. Then, a user inputs a start instruction by the input section 233 of the data processing unit 230 of the sample analyzer 2 in order to start the external accuracy management of the measuring unit 220. When the data processing unit 230 receives the instruction, the data processing unit 230 transmits a measurement start instruction to the measuring unit 220.

When receiving a signal of the measurement start instruction, the measuring unit 220 performs the primer reagent dispensing process, the enzyme reagent dispensing process, and the control solution dispensing process of dispensing the CK19 control solution of the sample container 22 into one cell section 66a of the detection cell 65 and dispensing the arabido control solution into the other cell section 66a. Thereafter, the measuring unit 220 amplifies the target nucleic acid (CK19) and the arabido in accordance with the LAMP method by increasing the liquid temperature in the detection cell 65 from about 20°C to about 65°C, and performs a detection process of detecting (monitoring) the liquid turbidity in each cell section 66a of the detection cell 65 in the amplification reaction in real time by the turbidity detection section 62.

When optical information (measurement data of CK19 and measurement data of arabido) is detected by the measuring unit 220, the optical information (measurement data) is analyzed by the data processing unit 230. In the analysis process, the amplification rise time of CK19, the number of copies of CK19, and the amplification rise time of arabido are calculated. The amplification rise times of CK19 and arabido are calculated as a time until the liquid turbidity obtained as optical information exceeds a predetermined value, and the number of copies of CK19 is calculated from the amplification rise time of CK19 on the basis of the calibration curve.

The above-described analysis result (measurement data) is transmitted to the first mail server 4 by the data processing unit 230. When receiving the measurement data, the first mail server 4 transmits the measurement data to the database server 5 and the measurement data is registered in the accuracy management database. In addition, the database server 5 subjects a large number of pieces of measurement data transmitted from a large number of the sample analyzers 2 which are installed in the respective facilities to the statistical process. In greater detail, on the basis of the measurement data transmitted from the sample analyzers 2 which are installed in a plurality of facilities, the average value for each day and the standard deviation 1SD are obtained. In addition, the database server 5 also obtains 2SD, which is two times the standard deviation 1SD, and 3SD, which is three times the standard deviation 1SD. The average value of the measurement data for each day, 1SD, 2SD, and 3SD are registered in the accuracy management database of the database server 5. In the accuracy management database, the measurement data which is obtained by measuring the control solution by a reference apparatus is also accumulated.

Furthermore, when receiving the measurement data, the database server 5 determines whether or not the sample measurement by the measuring unit is normal on the basis of the calculated average value and 1SD, 2SD or 3SD. In greater detail, the database server 5 determines whether or not the sample measurement is normal on the basis of the average value of the measurement data which was received over a certain period of past time (for example, 24 hours in the past) and the standard deviation 1SD, 2SD or 3SD. Each of the 1SD, the 2SD, and the 3SD can be a reference value for whether or not the received measurement data is normal. Which one of the 1SD, the 2SD, and the 3SD is used as the reference value is selected by each facility and the selected reference value is used in determination. Such a determination result is also registered in the accuracy management database.

When the accuracy management data (measurement data, statistical data and determination result) is registered in the accuracy management database, the database server 5 transmits the registered accuracy management data to the web server 8. The web server 8 stores the received accuracy management data in the database in the hard disk. Such accuracy management data can be viewed from the other computers (data processing unit 230 and client devices 9) by the web server 8.

Next, a user executes the preprocessing and the sample measurement using a tissue cut actually from a patient. In the sample measurement, the above-described tissue is set in the sample setting section 213 of the preprocessing unit 210. In addition, the user inputs a sample measurement start instruction by the input section 233 of the data processing unit 230 of the sample analyzer 2 in order to start the sample preprocessing. When receiving such a sample measurement start instruction (Step S111), the CPU 231a generates and transmits an e-mail for notifying of the start of sample measurement to the first mail server (Step S112). In the subject of this e-mail, the operation state code "4", showing the state in which the sample measurement is started, is included. Thereafter, the sample analyzer 2 executes the sample preprocessing and the sample measurement (Step S113).

The process in Step S113 will be described in detail. When the preprocessing unit 210 receives a signal of the measurement start instruction, the preprocessing section 211 subjects the sample to the process of adding a reagent for preprocessing and the homogenization process to prepare a sample for measurement. This sample for measurement is given to the measuring section 212 of the preprocessing unit 210 and the absorbance is measured. The absorbance measurement data is transmitted to the first mail server 4 by the data processing unit 230.

When receiving the absorbance measurement data, the first mail server 4 transmits the absorbance measurement data to the database server 5 and the measurement data is registered in the measurement result database. When the absorbance measurement data of the preprocessing is registered in the measurement result database, the database server 5 transmits the registered measurement data to the web server 8. The web server 8 stores the received measurement data in the database in the hard disk. Such measurement data can be viewed from the other computers (data processing unit 230 and client devices 9) by the web server 8.

When receiving a signal of the measurement start instruction, the measuring unit 220 performs the primer reagent dispensing process, the enzyme reagent dispensing process, and the solution dispensing process of dispensing the CK19 solution of the sample container 22 into one cell section 66a of the detection cell 65 and dispensing the arabido control solution into the other cell section 66a. Thereafter, the measuring unit 220 amplifies the target nucleic acid (CK19) and the arabido in accordance with the LAMP method by increasing the liquid temperature in the detection cell 65 from about 20°C to about 65°C, and performs a detection process of detecting (monitoring) the liquid turbidity in each cell section 66a of the detection cell 65 in the amplification reaction in real time by the turbidity detection section 62.

When optical information (measurement data of CK19 and measurement data of arabido) is detected by the measuring unit 220, the optical information (measurement data) is analyzed by the data processing unit 230. In the analysis process, the amplification rise time of CK19, the number of copies of CK19, and the amplification rise time of arabido are calculated. The amplification rise times of CK19 and arabido are calculated as a time until the liquid turbidity obtained as optical information exceeds a predetermined value, and the number of copies of CK19 is calculated from the amplification rise time of CK19 on the basis of the calibration curve.

The above-described analysis result (measurement data) is transmitted to the first mail server 4 by the data processing unit 230. When receiving the measurement data, the first mail server 4 transmits the measurement data to the database server 5 and the measurement data is registered in the measurement result database. When the measurement data is registered in the measurement result database, the database server 5 transmits the registered measurement data to the web server 8. The web server 8 stores the received measurement data in the database in the hard disk. Such measurement data can be viewed from the other computers (data processing unit 230 and client devices 9) by the web server 8.

When the above-described sample measurement ends, the CPU 231a generates and transmits an e-mail for notifying the end of sample measurement to the first mail server (Step S114) . In the subject of this e-mail, the operation state code "5", showing the state in which the sample measurement ends, is included.

When stopping the operation of the sample analyzer 2, a user operates the input section 233 of the data processing unit 230 and inputs a shutdown instruction. When receiving such a shutdown instruction (Step S115) , the CPU 231a generates and transmits an e-mail for notifying of the shutdown of the sample analyzer 2 to the first mail server (Step S116). In the subject of this e-mail, the operation state code "6", showing the state in which the measurement unit is exited, is included. In addition, the CPU 231a generates and transmits an e-mail including the operation history such as the number of suction operations of the pipette of the sample analyzer 2 to the first mail server (Step S117) . When the shutdown of the sample analyzer 2 is completed, the CPU 231a ends the process.

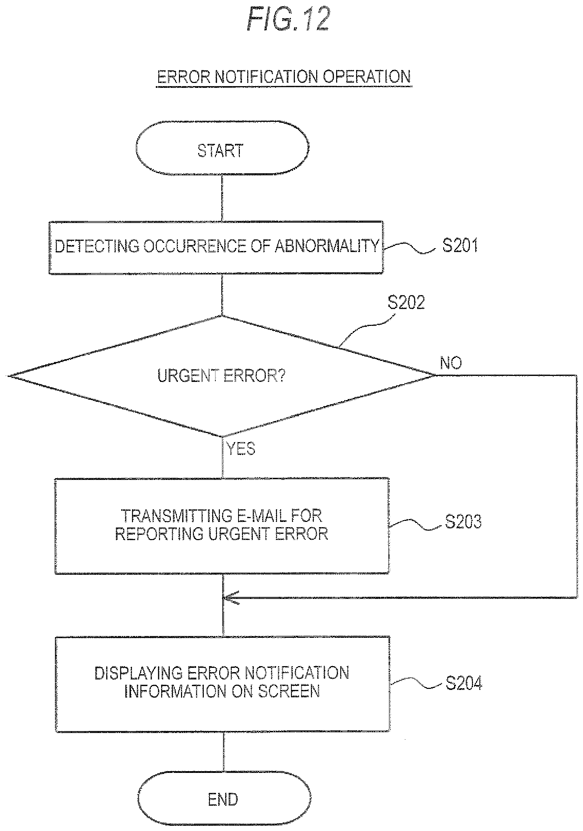

Next, an error notification operation of the sample analyzer 2 will be described. Fig. 12 is a flowchart showing the procedure of the error notification operation of the sample analyzer 2. When an abnormality occurs in the preprocessing unit 210 or the measuring unit 220, the sample analyzer 2 detect the occurrence of the abnormality by a sensor (Step S201). Among abnormalities, an abnormality such as cessation of the measurement is a severe abnormality by which the measurement cannot continue, whereby it is necessary to rapidly cope with it. The kinds of abnormality to be rapidly coped with (hereinafter, referred to as "urgent error") are stored on the hard disk 231d of the data processing unit 230. The CPU 231a determines whether or not the detected abnormality is an urgent error (Step S202) . When the abnormality is not an urgent error (NO in Step S202), a screen for notifying of the occurrence of the error is displayed on the image display section 232 of the data processing unit 230 (Step S204) , and the process ends.

On the other hand, when the detected abnormality is an urgent error (YES in Step S202), the CPU 231a generates and transmits an e-mail for notifying of the urgent error (Step S203) . The destination of this e-mail is a mail address for providing maintenance service, and the e-mail includes the model code of the sample analyzer, the apparatus ID of the sample analyzer, and the error code showing the detected abnormality in the subject. In addition, the body of the e-mail is left blank. When the e-mail is transmitted, the CPU 231a displays a screen for notifying of the occurrence of the error on the image display section 232 of the data processing unit 230 (Step S204), and ends the process.

### <Operation of Maintenance Management System>

Next, the operation of the maintenance management system 3 when the above-described e-mail is transmitted will be described. The e-mail which is transmitted to the mail address for providing maintenance service as the destination from the sample analyzer 2 is received by the first mail server 4 and stored in the mail box of the first mail server 4. The first mail server 4 extracts and transmits information such as the apparatus ID, the model code, the operation state code, and the urgent error code from the e-mail to the database server 5. The database server 5 stores the received data in the database DB or another database.

Here, the registration of the data by the database server 5 when the first mail server 4 receives an e-mail for reporting an operation state will be described. When receiving the apparatus ID, the model code, and the operation state code from the first mail server 4, the database server 5 generates a reception number of this data and stores a reception time. Next, the database server 5 registers the reception number acquired as described above, the reception time, the model code, the apparatus ID, and the operation state code as a new record in the database DB. In addition, at this time, no information is stored in the field F6 for technician, the field F7 for processing segment, and the field F8 for time and date of the visit in the record.

Next, the database server 5 determines whether or not the data received from the first mail server 4 is information to be notified to a technician. Hereinafter, the information to be notified to a technician will be described.

The sample analyzer 2 is used in intraoperative rapid diagnosis. Accordingly, when an abnormality occurs in the sample analyzer 2, it is required to immediately handle the abnormality. Therefore, the state in which the sample analyzer 2 is operating is thought as a preparation state in which the sample measurement is to be executed or a state in the course of the sample measurement, and thus when a technician stands by at this time, the technician can rapidly handle an abnormality even when the abnormality occurs in the sample analyzer 2. Accordingly, in the sample analyzing system 1 according to this embodiment, when an e-mail for reporting the above-described operation state of the sample analyzer 2 is received, the technician is notified of the fact that the report has been performed. In this manner, when the sample analyzer 2 is operating, a technician who can handle the trouble of the sample analyzer 2 can be secured. In addition, when an urgent error occurs in the sample analyzer 2, it is necessary to immediately cope with the error. Accordingly, in the sample analyzing system 1 according to this embodiment, when an e-mail for reporting the above-described urgent error of the sample analyzer 2 is received, a technician is notified of the fact that the report has been performed. That is, the operation state code of the sample analyzer 2 and the urgent error code are judged to be information to be notified to the technician. The operation history information which is transmitted at the shutdown of the sample analyzer 2 is not determined to include the information to be notified to the technician.

When receiving the operation history information, the database server 5 registers the operation history information in a database (not shown) and transmits the registered operation history information to the web server 8. The web server 8 stores the received operation history information in the database in the hard disk. Such operation history information can be viewed from the other computers (data processing unit 230 and client devices 9) by the web server 8.

When the database server 5 determines that the received information is the information to be notified to the technician, the database server 5 creates and transmits an e-mail including the information to the second mail server 6. This e-mail has the same configuration as that of the e-mail for reporting the above-described operation state of the sample analyzer 2 or urgent error, except that the destination is a mail address for reporting to the technician.

The e-mail transmitted from the database server 5 is received by the second mail server 6 and stored in the mail box of the second mail server 6. The second mail server 6 extracts and transmits information such as the apparatus ID, the model code, the operation state code, and the urgent error code from the e-mail to the management server 7.

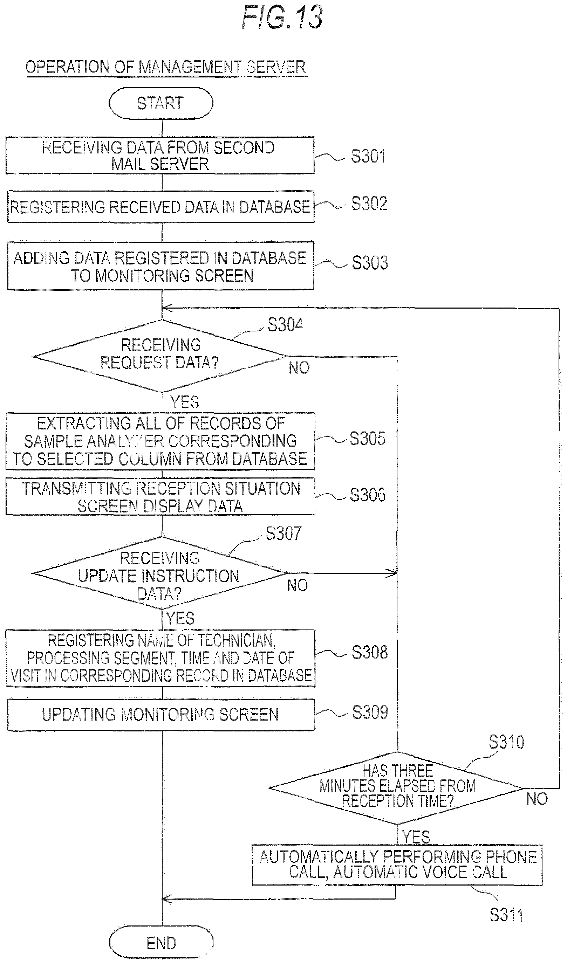

Fig. 13 is a flowchart showing the procedure of the operation of the management server 7. When receiving the data from the second mail server 6 (Step S301) , the CPU 710a of the management server 7 registers the received data in the database (step S302). Since the configuration of the database is the same as the above-described configuration of the database DB of the database server 5, the description thereof will be omitted.

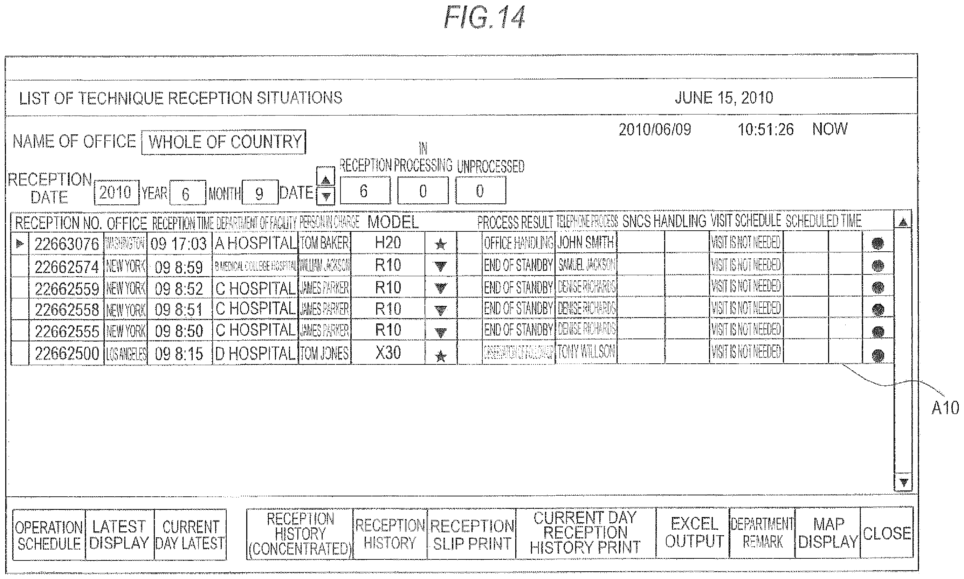

Next, the CPU 710a adds the data registered in the database to a monitoring screen which can be viewed from each client device 9 in common (Step S303). Fig. 14 is a diagram showing an example of the monitoring screen. The monitoring screen displays handling situations of technicians with respect to facilities to be handled by the technicians at that time in a list format. In an area A10 displaying the reception situations, the reception number, the office in charge of customers, the reception time, the department of the customer' s facility in which the sample analyzer is installed, the name of the technician in charge, the model code, the mark representing the kind of the situation occurring in the sample analyzer, the process result (result of the handling of the technician) , the name of person in charge for calling, the visit schedule, and the time and date of the visit are arranged as display items. The apparatus ID received by the management server 7 from the second mail server 6 is information which is set uniquely to each sample analyzer 2 . In a customer database (not shown) provided in the hard disk of the management server 7, the office in charge of customers, the department of the customer's facility in which the sample analyzer is installed, the model code, and the apparatus ID are recorded to be associated therewith. Such a management server 7 acquires the information of the office and the department of the customer's facility from the apparatus ID received from the second mail server, and thus displays the received information in the area A10.

In addition, one row of the area A10 corresponds to one sample analyzer. That is, the rows display information related to sample analyzers different from each other, respectively. For example, before the start-up of a sample analyzer 2, the information related to the sample analyzer 2 is not displayed in the area A10. In this state, when the sample analyzer 2 is started-up, an e-mail for reporting the start-up state is transmitted from the sample analyzer 2, and the model code and the apparatus ID of the sample analyzer 2, and the operation state code "C" showing the start-up state are received by the management server 7. In this manner, the row corresponding to the sample analyzer 2 is newly added in the area A10 of the monitoring screen at this time, and in the row, the information of the reception number, the office, the reception time, the department of facility, the model code, the mark representing the kind of the situation occurring in the sample analyzer, the process result, and the visit schedule is displayed. Here, as the mark representing the kind of the situation occurring in the sample analyzer, the marks ▼ and ★ are provided. The mark ▼ represents that the operation state report is received from the sample analyzer 2 (that is, the sample analyzer 2 is operating), and the mark ★ represents that the urgent error report is received from the sample analyzer 2 (that is, an urgent error occurs in the sample analyzer 2). That is, regarding the sample analyzer 2, when the operation state code is stored in the database, the management server 7 displays the mark **▼** and when the urgent error code is stored in the database, the management server 7 displays the mark ★. In addition, the marks ▼ and ★ are displayed by a red color when a technician does not handle the operation state report or the urgent error report of the sample analyzer, and the marks ▼ and ★ are displayed by a blue color when a technician has handled the operation state report or the urgent error report of the sample analyzer. In this manner, the technician can easily confirm whether the operation state report and the urgent error report are handled by just referring to the monitoring screen.

In addition, when an e-mail for reporting the operation state or an e-mail for reporting an urgent error is further transmitted from the sample analyzer 2 of which the apparatus information is displayed on the monitoring screen, the information which is included in this e-mail is registered as a new record of the database of the management server 7. However, a row corresponding to the record is not added to the monitoring screen, but the display of the row for the sample analyzer 2 is updated to new information. For example, in the case in which a report of the start-up state is received from the sample analyzer 2 and the information of this start-up state is displayed in the area A10 of the monitoring screen, when a report of the state in which the calibration curve measurement is started is received from the sample analyzer 2, the reception number and the reception time of the row is updated to a reception number and a reception time related to the newly received calibration curve report. In addition, in this state, when a further urgent error occurs in the sample analyzer 2 and an urgent error report is received from the sample analyzer 2, the reception number and the reception time of the row are updated to a reception number and a reception time related to the newly received calibration curve report, and the mark changes to the mark ★. In this manner, the information reflecting the new operation state of the sample analyzer 2 can be displayed without increasing rows.

Technicians can log into the management server 7 by using the client device 9. The above-described monitoring screen is displayed on all of the client devices 9 which log into the management server 7. Accordingly, each technician can recognize which sample analyzer 2 in which facility is operating by confirming the monitoring screen.

Technicians can select one row in the area A10 of the monitoring screen by operating the input section such as a mouse of the client device 9. In this manner, when one row in the area A10 of the monitoring screen is selected, request data for requesting specific information of the sample analyzer 2 corresponding to the row is transmitted to the management server 7. This request data includes information which specifies the row. When receiving such request data (YES in Step S304), the CPU 710a of the management server 7 extracts all of the records of the sample analyzer 2 corresponding to the row from the database (Step S305) . For example, when the apparatus ID of the sample analyzer corresponding to the selected row is "R0001", all of the records of the apparatus ID "R0001" are extracted from the database. In this manner, after extraction of the information from the database, the CPU 710a transmits reception situation screen display data to the client device 9 which is a transmission source of the request data (Step S306). When receiving such reception situation screen display data, the client device 9 displays a reception situation screen which to be described as follows.

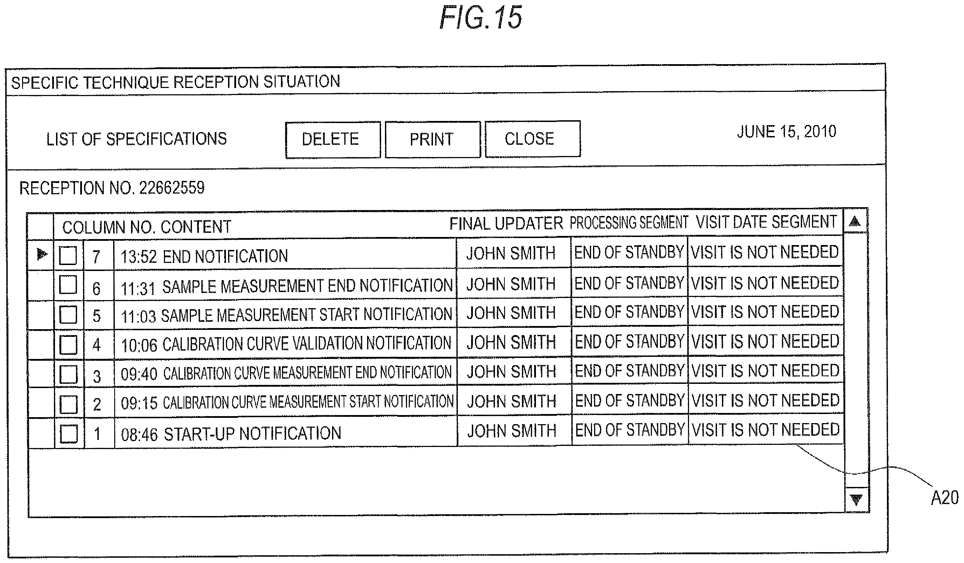

Fig. 15 is a diagram showing an example of a reception situation screen. The reception situation screen displays the information of the operation state reports which have been received from the sample analyzer 2 in a list format. The reception situation screen has an area A20, and in the area A20, the operation state reports which have been received from the start-up of the sample analyzer 2 are displayed. One row of the area A20 corresponds to one operation state report, and the pieces of operation state report information are displayed to be time-sequentially arranged so that new information is positioned on the upper side and old information is positioned on the lower side. Accordingly, technicians can know the current situation of the sample analyzer 2 by referring to the operation state report information at the top.

When a technician does not handle the operation state report, the reception time and the content of the notification are displayed in the reception situation screen, and the "final updater", "processing segment" and "visit date segment" are left blank. When a technician handles the sample analyzer 2, the technician inputs his name in the "final updater" and inputs "standby" or "end of standby" in the "processing segment". Here, the "end of standby" represents that "on that day, the standby before the client device 9 with respect to the sample analyzer 2 has ended", and "standby" represents that "the standby before the client device 9 with respect to the sample analyzer 2 continues". That is, the final updater "stands by" from when a report of the start-up state of the sample analyzer 2 is received to when a report of the end state of the sample analyzer 2 is received. In this manner, when a technician inputs the "final updater", "processing segment" and "visit date segment", the input information is transmitted to the management server 7 from the client device 9. When receiving such input information (updated instruction data) (YES in Step S307), the CPU 710a of the management server 7 registers the input name of the technician, processing segment, and time and date of the visit in the field for technician, the field for processing segment, and the field for time and date of visit, respectively (Step S308), updates the name of the technician, processing segment, and time and date of the visit in a row corresponding to the sample analyzer 2 in the area A10 of the monitoring screen, and changes the color of the mark ▼ or ★ in the row from red to blue (step S309) . In this manner, the monitoring screen is updated and then the CPU 710a ends the process.

In addition, when a row in which information related to an urgent error report is displayed is selected in the area A10 of the monitoring screen, the information showing the content of the error is displayed. In this manner, a technician can easily confirm what kind of error has occurred.

When a technician refers to the above-described monitoring screen or reception situation screen, it is possible to easily confirm that the sample analyzer is operating or that an urgent error occurs in the sample analyzer. In addition, in the case in which a technician monitors the operation situation of the sample analyzer 2, when an abnormality occurs in the sample analyzer 2 or when an urgent error occurs in the sample analyzer 2, the technician can access the web server 8 from the client device 9 and refer to the accuracy management result of the preprocessing unit or the measuring unit of the sample analyzer, the operation history of the sample analyzer, or the measurement data of the sample analyzer 2. In this manner, the technician can specifically examine the kind of the error which has occurred in the sample analyzer and the coping strategy.

When the request data is not received in Step S304 (NO in Step S304), or when the update instruction data is not received in Step S307 (NO in Step S307) , the CPU 710a determines whether or not three minutes (predetermined time) has elapsed from the reception time at which the operation state report information or the urgent error report information was received (Step S309) . When three minutes has not elapsed, the CPU 710a returns the process to Step S304. On the other hand, when three minutes (predetermined time) has elapsed from the reception time at which the operation state report information or the urgent error report information was received (YES in Step S309) , the CPU 710a automatically makes a call to a telephone 300, contacts a technician who can handle the sample analyzer 2, outputs an automatic voice message for instructing to stand by before the client device 9 (step S310) , and ends the process. The technician who hears the voice message through the telephone 300 contacts a technician who can handle the sample analyzer 2 so that the technician stands by before the client device 9. In this manner, the technician who can handle the sample analyzer 2 can be reliably secured.

### (Other Embodiments)

In the above-described embodiments, the sample analyzer 2 which reports the operation state is a nucleic acid amplification detector, but the invention is not limited thereto. A configuration may be employed in which a blood cell counter, a blood coagulation measurement apparatus, an immunoassay apparatus, a biochemical analyzer, a urine qualitative analyzer, or an in-urine physical component analyzer reports the operation state.

In addition, in the above-described embodiments, examples of the event related to the start of sample measurement include (1) an event related to the measurement of a patient sample, (2) an event related to the start-up of the sample analyzer, (3) an event related to the measurement of a standard sample for preparation of a calibration curve or accuracy management, and (4) an event related to the approval for a prepared calibration curve or the accuracy management result, but the invention is not limited thereto. For example, as a fifth type of the eventrelated to the start of sample measurement, an event occurring by a temporal factor may be included. For example, elapse of a predetermined time required to prepare the start of sample measurement from the start-up of the sample analyzer by a user may be included as an event.

In addition, in the above-described embodiments, as an example of (1) the event related to the measurement of a patient sample, the reception of an instruction for starting the measurement of a patient sample from a user by the input section 233 has been provided, but the invention is not limited thereto. For example, the registration of a measurement order by the input section 233, the detection of the setting of a reagent container by a user by a sensor, the detection of the setting of a patient sample by a user by a sensor, or the like may be included as an event.

In addition, in the above-described embodiments, as an example of (2) the event related to the start-up of the sample analyzer, the shift to a standby state of the measuring unit 220 has been provided, but the invention is not limited thereto. For example, the power-on of the measuring unit 220, the shift to a standby state of the information processing unit 230, the start-up of an application program for control of the measuring unit 220, which is stored in the information processing unit 230, or the like may be included as an event.

In addition, in the above-described embodiments, as an example of (3) the event related to the measurement of a standard sample for preparation of a calibration curve or accuracy management, the reception of a calibrator measurement start instruction from a user by the input section 233 has been provided, but the invention is not limited thereto. For example, the completion of the measurement of the calibrator, the detection of the setting of the calibrator by a user by a sensor, or the like may be included as an event.

In addition, in the above-described embodiments, as an example of (4) the event related to the approval for a prepared calibration curve or the accuracy management result, the reception of a validation for the calibration curve from a user by the input section 233 has been provided, but the invention is not limited thereto. For example, the display of a screen for receiving a validation for the calibration curve or the accuracy management result by the information processing unit 230 may be included as an event.

In addition, in the above-described embodiments, the management apparatus receives a report in any of the cases when the events (1) to (4) occur. However, a configuration may be employed in which the reporting to the management apparatus is executed only when some of the above-described plurality of events occurs.

In addition, in the above-described embodiments, the configuration has been described in which the maintenance management system 3 includes the first mail server 4, the database server 5, the second mail server 6, the management server 7, the web server 8, and the client devices 9, 9, ..., but the invention is not limited thereto. A configuration may be employed in which the functions of the first mail server 4, the database server 5, the second mail server 6, the management server 7, and the web server 8 are realized by one server computer. A configuration may also be employed in which a distributing system in which the function of the management server 7 is constituted of two or more computers is provided.

In addition, the configuration has been described in which the monitoring screen and the reception situation screen are displayed on the image display section of the client device 9, but the invention is not limited thereto. A configuration may be employed in which the monitoring screen and the reception situation screen are displayed on the image display section of the management server 7. A configuration may also be employed in which the client device 9 of the management server 7 is not provided and the functions of the management server 7 and the client device 9 are realized by one computer to be used by a technician to display the monitoring screen and the reception situation screen on the image display section of the computer.

## Claims

1. A clinical sample analyzing system comprising:
a clinical sample analyzer (2) configured to analyze a clinical sample, wherein the clinical sample analyzer is suitable to be installed in a facility of an user; and
a management apparatus (7) which is able to perform data communication with the clinical sample analyzer and is suitable to be installed in a facility different from the facility of the user,
wherein the clinical sample analyzer is configured to automatically transmit report data showing an operation situation of the clinical sample analyzer to the management apparatus when a predetermined event related to an initiation of a sample measurement occurs, and
the management apparatus is configured to output a notification of the operation situation of the clinical sample analyzer when receiving the report data from the clinical sample analyzer.

2. The clinical sample analyzing system according to Claim 1, wherein when a first event related to an initiation of a sample measurement occurs, the clinical sample analyzer (2) is configured to transmit first report data showing the occurrence of the first event to the management apparatus (7), and
when a second event related to an initiation of a sample measurement occurs after the occurrence of the first event, the clinical sample analyzer is configured to transmit second report data showing the occurrence of the second event to the management apparatus.

3. The clinical sample analyzing system according to Claim 1 or 2, wherein the predetermined event includes at least one of:
an event related to a measurement of a patient sample;
an event related to the start-up of the clinical sample analyzer (2);
an event related to the measurement of a standard sample for preparation of a calibration curve or accuracy management;
and
an event related to the approval for a prepared calibration curve or an accuracy management result.

4. The clinical sample analyzing system according to any one of Claims 1 to 3, wherein the clinical sample analyzer (2) is configured to automatically transmit a measurement completion report for reporting a completion of a sample measurement to the management apparatus (7) when the sample measurement is completed, and
the management apparatus is configured to output a notification of the completion of a sample measurement when receiving the measurement completion report.

5. The clinical sample analyzing system according to any one of Claims 1 to 4, wherein the clinical sample analyzer (2) is configured to automatically transmit an operation termination report for reporting a termination of the operation of the clinical sample analyzer to the management apparatus (7) when executing a termination operation of the clinical sample analyzer, and
the management apparatus is configured to output a notification of the termination of the operation of the clinical sample analyzer when receiving the operation termination report.

6. The clinical sample analyzing system according to any one of Claims 1 to 5, wherein the clinical sample analyzer (2) is configured to transmit an analysis result to the management apparatus (7) when completing the analysis of the clinical sample, and
the management apparatus is able to output the received analysis result.

7. The clinical sample analyzing system according to any one of Claims 1 to 6, wherein the clinical sample analyzer (2) is configured to automatically transmit abnormality report data showing an abnormality to the management apparatus (7) when the abnormality occurs in the clinical sample analyzer, and
the management apparatus is configured to output the occurrence of the abnormality in the clinical sample analyzer when receiving the abnormality report data.

8. The clinical sample analyzing system according to Claim 7, wherein the abnormality report data includes information showing the content of the abnormality occurring in the clinical sample analyzer (2), and
the management apparatus (7) is configured to output the information showing the content of the abnormality in the clinical sample analyzer when receiving the abnormality report data.

9. The clinical sample analyzing system according to any one of Claims 1 to 8, wherein after output of the notification, the management apparatus (7) is configured to receive a confirmation input from a person receiving the notification, and when receiving a confirmation input, is configured to record a reception of a confirmation input.

10. The clinical sample analyzing system according to Claim 9, wherein the management apparatus (7) is connected to a phone line, and when a predetermined time elapses from the execution of the notification without reception of the confirmation input, the management apparatus is configured to perform a phone call to a predetermined phone number in order to prompt the confirmation of the notification.

11. The clinical sample analyzing system according to any one of Claims 1 to 10, wherein the clinical sample analyzer (2) is configured to amplify a target nucleic acid in a tissue collected from a patient and to detect an amplified target nucleic acid.

12. A clinical sample analyzer (2) which is suitable to be installed in a facility of an user, the analyzer comprising:
means (231g) for performing data communication with a management apparatus (7) which is suitable to be installed in a facility different from the facility of the user,
wherein the means for performing data communication is configured to automatically transmit report data showing an operation situation of the clinical sample analyzer to a management apparatus (7) when a predetermined event related to an initiation of a sample measurement occurs.

13. A management apparatus (7) which is suitable to be installed in a facility different from a facility of an user in which a clinical sample analyzer (2) is to be installed, the management apparatus capable of performing data communication with the clinical sample analyzer and comprising:
means (710g) for receiving, when a predetermined event related to an initiation of a sample measurement occurs, report data showing an operation situation of the clinical sample analyzer which is transmitted from the clinical sample analyzer in order to report the occurrence of the predetermined event; and
means (710h) for outputting a notification of the operation situation of the clinical sample analyzer when the report data is received by the receiving section.

14. A method of managing a clinical sample analyzer (2), the method comprising:
a transmitting step of automatically transmitting report data showing an operation situation of the clinical sample analyzer by the clinical sample analyzer when a predetermined event related to an initiation of a sample measurement occurs in the clinical sample analyzer which is installed in a facility of an user; and
an output step of outputting a notification of the operation situation of the clinical sample analyzer when a management apparatus (7) receives the report data, wherein the management apparatus is able to perform data communication with the clinical sample analyzer and is installed in a facility different from the facility of an user.

## Patentansprüche

1. Klinisches Probenanalysesystem, umfassend:
ein klinisches Probenanalysegerät (2), das zum Analysieren einer klinischen Probe konfiguriert ist, wobei das klinische Probenanalysegerät geeignet ist, in einer Anlage eines Benutzers installiert zu werden; und
eine Verwaltungseinrichtung (7), die in der Lage ist, eine Datenkommunikation mit dem klinischen Probenanalysegerät durchzuführen, und die geeignet ist, in einer Anlage installiert zu werden, die sich von der Anlage des Benutzers unterscheidet,
wobei das klinische Probenanalysegerät so konfiguriert ist, dass es automatisch Berichtsdaten, die eine Betriebssituation des klinischen Probenanalysegeräts zeigen, an die Verwaltungseinrichtung überträgt, wenn ein vorbestimmtes Ereignis in Bezug auf eine Einleitung einer Probenmessung auftritt, und
die Verwaltungseinrichtung konfiguriert ist, um eine Benachrichtigung über die Betriebssituation des klinischen Probenanalysegeräts auszugeben, wenn sie die Berichtsdaten vom klinischen Probenanalysegerät empfängt.

2. Klinisches Probenanalysesystem nach Anspruch 1, wobei das klinische Probenanalysegerät (2) so konfiguriert ist, dass es bei Auftreten eines ersten Ereignisses in Bezug auf eine Einleitung einer Probenmessung erste Berichtsdaten, die das Auftreten des ersten Ereignisses zeigen, an die Verwaltungseinrichtung (7) überträgt, und
wenn ein zweites Ereignis in Bezug auf eine Einleitung einer Probenmessung nach dem Auftreten des ersten Ereignisses auftritt, das klinische Probenanalysegerät so konfiguriert ist, dass es zweite Berichtsdaten, die das Auftreten des zweiten Ereignisses zeigen, an die Verwaltungseinrichtung überträgt.

3. Klinisches Probenanalysesystem nach Anspruch 1 oder 2, wobei das vorbestimmte Ereignis mindestens eines der folgenden einschließt:
ein Ereignis in Bezug auf eine Messung einer Patientenprobe;
ein Ereignis in Bezug auf die Inbetriebnahme des klinischen Probenanalysegeräts (2);
ein Ereignis in Bezug auf die Messung einer Standardprobe zur Erstellung einer Kalibrierkurve oder zur Genauigkeitsverwaltung; und
ein Ereignis in Bezug auf die Freigabe für eine erstellte Kalibrierkurve oder ein Genauigkeitsverwaltungsergebnis.

4. Klinisches Probenanalysesystem nach einem der Ansprüche 1 bis 3, wobei das klinische Probenanalysegerät (2) so konfiguriert ist, dass es automatisch einen Messungsabschlussbericht zur Meldung eines Abschlusses einer Probenmessung an die Verwaltungseinrichtung (7) überträgt, wenn die Probenmessung abgeschlossen ist, und
die Verwaltungseinrichtung so konfiguriert ist, dass sie eine Benachrichtigung über den Abschluss einer Probenmessung ausgibt, wenn sie den Messungsabschlussbericht empfängt.

5. Klinisches Probenanalysesystem nach einem der Ansprüche 1 bis 4, wobei das klinische Probenanalysegerät (2) so konfiguriert ist, dass es automatisch einen Betriebsbeendigungsbericht zur Meldung einer Beendigung des Betriebs des klinischen Probenanalysegeräts an die Verwaltungseinrichtung (7) überträgt, wenn es einen Beendigungsvorgang des klinischen Probenanalysegeräts ausführt, und
die Verwaltungseinrichtung so konfiguriert ist, dass sie eine Benachrichtigung über die Beendigung des Betriebs des klinischen Probenanalysegeräts ausgibt, wenn sie den Betriebsbeendigungsbericht empfängt.

6. Klinisches Probenanalysesystem nach einem der Ansprüche 1 bis 5, wobei das klinische Probenanalysegerät (2) so konfiguriert ist, dass es bei Abschluss der Analyse der klinischen Probe ein Analyseergebnis an die Verwaltungseinrichtung (7) überträgt, und
die Verwaltungseinrichtung in der Lage ist, das empfangene Analyseergebnis auszugeben.

7. Klinisches Probenanalysesystem nach einem der Ansprüche 1 bis 6, wobei das klinische Probenanalysegerät (2) so konfiguriert ist, dass es automatisch Anomalie-Berichtsdaten, die eine Anomalie zeigen, an die Verwaltungseinrichtung (7) überträgt, wenn die Anomalie in dem klinischen Probenanalysegerät auftritt, und
die Verwaltungseinrichtung ist so konfiguriert, dass sie das Auftreten der Anomalie im klinischen Probenanalysegerät ausgibt, wenn sie die Anomalie-Berichtsdaten empfängt.

8. Klinisches Probenanalysesystem nach Anspruch 7, wobei die Anomalie-Berichtsdaten Informationen einschließen, die den Inhalt der in dem klinischen Probenanalysegerät (2) auftretenden Anomalie zeigen, und
die Verwaltungseinrichtung (7) so konfiguriert ist, dass sie die Informationen ausgibt, die den Inhalt der Anomalie im klinischen Probenanalysegerät zeigen, wenn sie die Anomalie-Berichtsdaten empfängt.

9. Klinisches Probenanalysesystem nach einem der Ansprüche 1 bis 8, wobei die Verwaltungseinrichtung (7) so konfiguriert ist, dass sie nach Ausgabe der Benachrichtigung eine Bestätigungseingabe von einer die Benachrichtigung empfangenden Person empfängt, und bei Empfang einer Bestätigungseingabe so konfiguriert ist, dass sie einen Empfang einer Bestätigungseingabe aufzeichnet.

10. Klinisches Probenanalysesystem nach Anspruch 9, wobei die Verwaltungseinrichtung (7) mit einer Telefonleitung verbunden ist, und wenn eine vorbestimmte Zeit von der Ausführung der Benachrichtigung ohne Empfang der Bestätigungseingabe verstreicht, die Verwaltungseinrichtung so konfiguriert ist, dass sie einen Telefonanruf an eine vorbestimmte Telefonnummer durchführt, um die Bestätigung der Benachrichtigung zu veranlassen.

11. Klinisches Probenanalysesystem nach einem der Ansprüche 1 bis 10, wobei das klinische Probenanalysegerät (2) so konfiguriert ist, dass es eine Zielnukleinsäure in einem von einem Patienten gesammelten Gewebe amplifiziert und eine amplifizierte Zielnukleinsäure nachweist.

12. Klinisches Probenanalysegerät (2), das geeignet ist, in einer Anlage eines Benutzers installiert zu werden, wobei das Analysegerät Folgendes umfasst:
Mittel (231g) zur Durchführung einer Datenkommunikation mit einer Verwaltungseinrichtung (7), die geeignet ist, in einer Anlage installiert zu werden, die sich von der Anlage des Benutzers unterscheidet,
wobei das Mittel zur Durchführung der Datenkommunikation so konfiguriert ist, dass es automatisch Berichtsdaten, die eine Betriebssituation des klinischen Probenanalysegeräts zeigen, an eine Verwaltungseinrichtung (7) überträgt, wenn ein vorbestimmtes Ereignis in Bezug auf eine Einleitung einer Probenmessung auftritt.

13. Verwaltungseinrichtung (7), die geeignet ist, in einer Anlage installiert zu werden, die sich von einer Anlage eines Benutzers unterscheidet, in der ein klinisches Probenanalysegerät (2) installiert werden soll, wobei die Verwaltungseinrichtung in der Lage ist, eine Datenkommunikation mit dem klinischen Probenanalysegerät durchzuführen, und Folgendes umfasst:
Mittel (710g) zum Empfangen, wenn ein vorbestimmtes Ereignis in Bezug auf eine Einleitung einer Probenmessung auftritt, von Berichtsdaten, die eine Betriebssituation des klinischen Probenanalysegeräts zeigen, die von dem klinischen Probenanalysegerät übertragen werden, um das Auftreten des vorbestimmten Ereignisses zu melden; und
Mittel (710h) zur Ausgabe einer Benachrichtigung über die Betriebssituation des klinischen Probenanalysegerätes, wenn die Berichtsdaten vom empfangenden Bereich empfangen werden.

14. Verfahren zur Verwaltung eines klinischen Probenanalysegeräts (2), wobei das Verfahren Folgendes umfasst:
einen Übertragungsschritt zur automatischen Übertragung von Berichtsdaten, die eine Betriebssituation des klinischen Probenanalysegeräts zeigen, durch das klinische Probenanalysegerät, wenn ein vorbestimmtes Ereignis in Bezug auf eine Einleitung einer Probenmessung in dem klinischen Probenanalysegerät auftritt, das in einer Anlage eines Benutzers installiert ist; und
einen Ausgabeschritt zur Ausgabe einer Benachrichtigung über die Betriebssituation des klinischen Probenanalysegeräts, wenn eine Verwaltungseinrichtung (7) die Berichtsdaten empfängt, wobei die Verwaltungseinrichtung in der Lage ist, eine Datenkommunikation mit dem klinischen Probenanalysegerät durchzuführen und in einer Anlage installiert ist, die sich von der Anlage eines Benutzers unterscheidet.

## Revendications

1. Système d'analyse d'échantillons cliniques comprenant :
un analyseur d'échantillons cliniques (2) configuré pour analyser un échantillon clinique, dans lequel l'analyseur d'échantillons cliniques est approprié pour être installé dans une installation d'un utilisateur ; et
un appareil de gestion (7) qui est apte à effectuer une communication de données avec l'analyseur d'échantillons cliniques et est approprié pour être installé dans une installation différente de l'installation de l'utilisateur,
dans lequel l'analyseur d'échantillons cliniques est configuré pour transmettre automatiquement des données de rapport montrant une situation d'opération de l'analyseur d'échantillons cliniques à l'appareil de gestion lorsqu'un événement prédéterminé relatif à un déclenchement d'une mesure d'échantillon se produit, et
l'appareil de gestion est configuré pour délivrer en sortie une notification de la situation d'opération de l'analyseur d'échantillons cliniques lorsqu'il reçoit les données de rapport à partir de l'analyseur d'échantillons cliniques.

2. Système d'analyse d'échantillons cliniques selon la revendication 1, dans lequel lorsqu'un premier événement relatif à un déclenchement d'une mesure d'échantillon se produit, l'analyseur d'échantillons cliniques (2) est configuré pour transmettre des premières données de rapport montrant l'occurrence du premier événement à l'appareil de gestion (7), et
lorsqu'un second événement relatif à un déclenchement d'une mesure d'échantillon se produit après l'occurrence du premier événement, l'analyseur d'échantillons cliniques est configuré pour transmettre des secondes données de rapport montrant l'occurrence du second événement à l'appareil de gestion.

3. Système d'analyse d'échantillons cliniques selon la revendication 1 ou 2, dans lequel l'événement prédéterminé inclut au moins l'un de :
un événement relatif à une mesure d'un échantillon de patient ;
un événement relatif au démarrage de l'analyseur d'échantillons cliniques (2) ;
un événement relatif à la mesure d'un échantillon étalon pour la préparation d'une courbe d'étalonnage ou d'une gestion d'exactitude ;
et
un événement relatif à l'approbation pour une courbe d'étalonnage préparée ou un résultat de gestion d'exactitude.

4. Système d'analyse d'échantillons cliniques selon l'une quelconque des revendications 1 à 3, dans lequel l'analyseur d'échantillons cliniques (2) est configuré pour transmettre automatiquement un rapport d'achèvement de mesure pour rapporter un achèvement d'une mesure d'échantillon à l'appareil de gestion (7) lorsque la mesure d'échantillon est achevée, et
l'appareil de gestion est configuré pour délivrer en sortie une notification de l'achèvement d'une mesure d'échantillon lorsqu'il reçoit le rapport d'achèvement de mesure.

5. Système d'analyse d'échantillons cliniques selon l'une quelconque des revendications 1 à 4, dans lequel l'analyseur d'échantillons cliniques (2) est configuré pour transmettre automatiquement un rapport d'arrêt d'opération pour rapporter un arrêt de l'opération de l'analyseur d'échantillons cliniques à l'appareil de gestion (7) lorsqu'il exécute une opération d'arrêt de l'analyseur d'échantillons cliniques, et
l'appareil de gestion est configuré pour délivrer en sortie une notification de l'arrêt de l'opération de l'analyseur d'échantillons cliniques lorsqu'il reçoit le rapport d'arrêt d'opération.

6. Système d'analyse d'échantillons cliniques selon l'une quelconque des revendications 1 à 5, dans lequel l'analyseur d'échantillons cliniques (2) est configuré pour transmettre un résultat d'analyse à l'appareil de gestion (7) lorsqu'il achève l'analyse de l'échantillon clinique, et
l'appareil de gestion est apte à délivrer en sortie le résultat d'analyse reçu.

7. Système d'analyse d'échantillons cliniques selon l'une quelconque des revendications 1 à 6, dans lequel l'analyseur d'échantillons cliniques (2) est configuré pour transmettre automatiquement des données de rapport d'anomalie montrant une anomalie à l'appareil de gestion (7) lorsque l'anomalie se produit dans l'analyseur d'échantillons cliniques, et
l'appareil de gestion est configuré pour délivrer en sortie l'occurrence de l'anomalie dans l'analyseur d'échantillons cliniques lorsqu'il reçoit les données de rapport d'anomalie.

8. Système d'analyse d'échantillons cliniques selon la revendication 7, dans lequel les données de rapport d'anomalie incluent des informations montrant la teneur de l'anomalie se produisant dans l'analyseur d'échantillons cliniques (2), et
l'appareil de gestion (7) est configuré pour délivrer en sortie les informations montrant la teneur de l'anomalie dans l'analyseur d'échantillons cliniques lorsqu'il reçoit les données de rapport d'anomalie.

9. Système d'analyse d'échantillons cliniques selon l'une quelconque des revendications 1 à 8, dans lequel après la délivrance en sortie de la notification, l'appareil de gestion (7) est configuré pour recevoir une entrée de confirmation provenant d'une personne recevant la notification, et lorsqu'il reçoit une entrée de confirmation, est configuré pour enregistrer une réception d'une entrée de confirmation.

10. Système d'analyse d'échantillons cliniques selon la revendication 9, dans lequel l'appareil de gestion (7) est connecté à une ligne téléphonique, et lorsqu'un temps prédéterminé s'écoule à partir de l'exécution de la notification sans réception de l'entrée de confirmation, l'appareil de gestion est configuré pour effectuer un appel téléphonique vers un numéro de téléphone prédéterminé afin d'inviter la confirmation de la notification.

11. Système d'analyse d'échantillons cliniques selon l'une quelconque des revendications 1 à 10, dans lequel l'analyseur d'échantillons cliniques (2) est configuré pour amplifier un acide nucléique cible dans un tissu collecté à partir d'un patient et pour détecter un acide nucléique cible amplifié.

12. Analyseur d'échantillons cliniques (2) qui est approprié pour être installé dans une installation d'un utilisateur, l'analyseur comprenant :
un moyen (231g) pour effectuer une communication de données avec un appareil de gestion (7) qui est approprié pour être installé dans une installation différente de l'installation de l'utilisateur,
dans lequel le moyen pour effectuer une communication de données est configuré pour transmettre automatiquement des données de rapport montrant une situation d'opération de l'analyseur d'échantillons cliniques à un appareil de gestion (7) lorsqu'un événement prédéterminé relatif à un déclenchement d'une mesure d'échantillon se produit.

13. Appareil de gestion (7) qui est approprié pour être installé dans une installation différente d'une installation d'un utilisateur dans laquelle un analyseur d'échantillons cliniques (2) doit être installé, l'appareil de gestion étant capable d'effectuer une communication de données avec l'analyseur d'échantillons cliniques et comprenant :
un moyen (710g) pour recevoir, lorsqu'un événement prédéterminé relatif à un déclenchement d'une mesure d'échantillon se produit, des données de rapport montrant une situation d'opération de l'analyseur d'échantillons cliniques qui sont transmises à partir de l'analyseur d'échantillons cliniques afin de rapporter l'occurrence de l'événement prédéterminé ; et
un moyen (710h) pour délivrer en sortie une notification de la situation d'opération de l'analyseur d'échantillons cliniques lorsque les données de rapport sont reçues par la section réceptrice.

14. Procédé de gestion d'un analyseur d'échantillons cliniques (2), le procédé comprenant :
une étape de transmission consistant à transmettre automatiquement des données de rapport montrant une situation d'opération de l'analyseur d'échantillons cliniques par l'analyseur d'échantillons cliniques lorsqu'un événement prédéterminé relatif à un déclenchement d'une mesure d'échantillon se produit dans l'analyseur d'échantillons cliniques qui est installé dans une installation d'un utilisateur ; et
une étape de délivrance en sortie consistant à délivrer en sortie une notification de la situation d'opération de l'analyseur d'échantillons cliniques lorsqu'un appareil de gestion (7) reçoit les données de rapport, dans lequel
l'appareil de gestion est apte à effectuer une communication de données avec l'analyseur d'échantillons cliniques et est installé dans une installation différente de l'installation d'un utilisateur.
